# FASCICULE DE BREVET EUROPEEN

(11) **EP 1 370 550 B1**
(45) Date de publication et mention de la délivrance du brevet: **23.05.2007**
(21) Numéro de dépôt: 02722329.6
(22) Date de dépôt: 11.03.2002
(51) Int. Cl.: C07D 401/06, C07D 417/14, C07D 401/14, C07D 409/14, A61K 31/47

(54) **DERIVES DE LA QUINOLYL PROPYL PIPERIDINE, LEUR PREPARATION ET LES COMPOSITIONS QUI LES CONTIENNENT**
DERIVATE VON QUINOLYL-PROPYL-PIPERIDINE, DEREN HERSTELLUNG UND DIE SIE ENTHALTENDEN ZUSAMENSETZUNGEN
QUINOLYL PROPYL PIPERIDINE DERIVATIVES, THE PREPARATION THEREOF AND COMPOSITIONS CONTAINING SAME

(30) Priorité: 13.03.2001 FR 0103374
(43) Date de publication de la demande: 17.12.2003
(73) Titulaire: NOVEXEL, 93230 Romainville (FR)
(72) Inventeur: BACQUE, Eric, F-91190 GIF SUR YVETTE (FR); MIGNANI, Serge, F-92290 CHATENAY MALABRY (FR); MALLERON, Jean-Luc, F-91460 MARCOUSSIS (FR); TABART, Michel, F-91290 LA NORVILLE (FR); EVERS, Michel, F-94510 LA QUEUE EN BRIE (FR); VIVIANI, Fabrice, F-95380 LOUVRES (FR); EL-AHMAD, Youssef, F-94000 CRETEIL (FR); MUTTI, Stéphane, F-94170 LE PERREUX SUR MARNE (FR); DAUBIE, Christophe, F-75005 PARIS (FR)
(74) Mandataire: Hirsch & Associés
(86) Numéro de dépôt international: PCT/FR2002/000851
(87) Numéro de publication internationale: WO 2002/072572

(56) Documents cités:
- WO-A-00/43383
- WO-A-99/37635

## Description

La présente invention concerne des dérivés de quinolyl propyl pipéridine de formule générale : 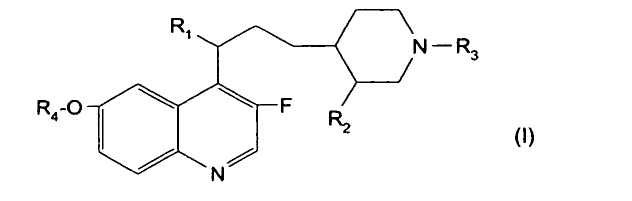 qui sont actifs comme antimicrobiens. L'invention concerne également leur préparation et les compositions les contenant.

Dans les demandes de brevet WO 99/37635 et WO 00/43383 ont été décrits des dérivés de quinolyl propyl pipéridine antimicrobiens, de formule générale : 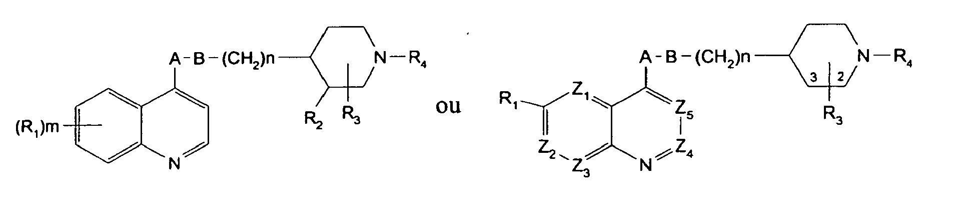 dans laquelle le radical R₁ est notamment alcoxy (C1-6), R₂ est hydrogène, R₃ est en position -2 ou -3 et représente alcoyle (C1-6) pouvant être éventuellement substitué par 1 à 3 substituants choisis parmi thiol, halogène, alcoylthio, trifluorométhyl, carboxy, alcoyloxycarbonyle, alcoylcarbonyle, alcènyloxycarbonyle, alcènylcarbonyle, hydroxy éventuellement substitué par alcoyle ..., R₄ est un groupe -CH₂-R₅ pour lequel R₅ est selectionné parmi alcoyle hydroxyalcoyle, alcènyle, alcynyle, tétrahydrofuryle, phénylalcoyle éventuellement substitué, phénylalcényle éventuellement substitué, hétéroarylalcoyle éventuellement substitué, hétéroaroyle éventuellement substitué ..., n est 0 à 2, m est 1 ou 2 et A et B sont notamment oxygène, soufre, sulfinyle, sulfonyle, NR₁₁, CR₆R₇ pour lequel R₆ et R₇ représentent H, thiol, alcoylthio, halo, trifluorométhyle, alcènyle, alcènylcarbonyle, hydroxy, amino, et Z₁ à Z₅ sont N ou CR₁ₐ ...

Dans la demande de brevet européen EP30044 ont été décrits des dérivés de quinoléine utiles comme cardiovasculaires, répondant à la formule générale : 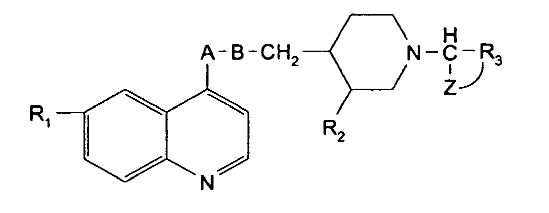 dans laquelle R₁ est notamment alcoyloxy, A-B est -CH₂-CH₂-, -CHOH-CH₂-, -CH₂-CHOH-, -CH₂-CO- ou -CO-CH₂-, R₁ est H, OH ou alcoyloxy, R₂ est éthyle ou vinyle, R₃ est notamment alcoyle, hydroxyalcoyle, cycloalcoyle, hydroxy, alcènyle, alcynyle, tétrahydrofuryle, phénylalcoyle, diphénylalcoyle éventuellement substitué, phénylalcényle éventuellement substitué, benzoyl ou benzoylalcoyle éventuellement substitué, hétéroaryle ou hétéroarylalcoyle éventuellement substitué et Z est H ou alcoyle ou forme avec R₃ un radical cycloalcoyle.

Il a maintenant été trouvé, et c'est ce qui fait l'objet de la présente invention, que les produits de formule générale (I) pour lesquels :
R₁ est un atome d'hydrogène ou d'halogène ou un radical hydroxy, amino, alcoylamino, dialcoylamino, hydroxyamino, alcoyloxyamino ou alcoyl alcoyloxy amino,
R₂ représente un radical carboxy, carboxyméthyle ou hydroxyméthyle,
R₃ représente un radical alcoyle (1 à 6 atomes de carbone) substitué par un radical phénylthio pouvant lui même porter 1 à 4 substituants [choisis parmi halogène, hydroxy, alcoyle, alcoyloxy, trifluorométhyle, trifluorométhoxy, carboxy, alcoyloxycarbonyle, cyano ou amino], par un radical cycloalcoylthio dont la partie cyclique contient 3 à 7 chaînons, ou par un radical hétérocyclylthio aromatique de 5 à 6 chaînons comprenant 1 à 4 hétéroatomes choisis parmi l'azote, l'oxygène ou le soufre et éventuellement lui même substitué [par halogène, hydroxy, alcoyle, alcoyloxy, trifluorométhyle, trifluorométhoxy, oxo, carboxy, alcoyloxycarbonyle, cyano ou amino] et
R₄ représente un radical alcoyle (contenant 1 à 6 atomes de carbone), alcényl-CH₂- ou alcynyl-CH₂- (dont les parties alcényle ou alcynyle contiennent 2 à 6 atomes de carbone), cycloalcoyle ou cycloalcoyl alcoyle (dont la partie cycloalcoyle contient 3 à 8 atomes de carbone)
sous leurs formes diastéréoisomères ou leurs mélanges et/ou sous leurs formes cis ou trans, ainsi que leurs sels, sont de puissants agents antibactériens.

Il est entendu que les radicaux et portions alcoyle sont en chaîne droite ou ramifiée et contiennent (sauf mention spéciale) 1 à 4 atomes de carbone, et que dans l'alternative où R₁ repésente un atome d'halogène ou lorsque R₃ porte un substituant halogène, celui-ci peut être choisi parmi fluor, chlore, brome ou iode. De préférence le fluor.

Dans la formule générale ci-dessus, lorsque R₃ porte un substituant hétérocyclyle aromatique, ce dernier peut être choisi parmi thiényle, furyle, pyrrolyle, imidazolyle, thiazolyle, oxazolyle, thiadiazolyle, oxadiazolyle, tétrazolyle, pyridyle, pyridazinyle, pyrazinyle, pyrimidinyle.

Selon l'invention, les produits de formule générale (I) peuvent être obtenus par condensation de la chaîne R₃ sur le dérivé de quinolyl propyl pipéridine de formule générale : 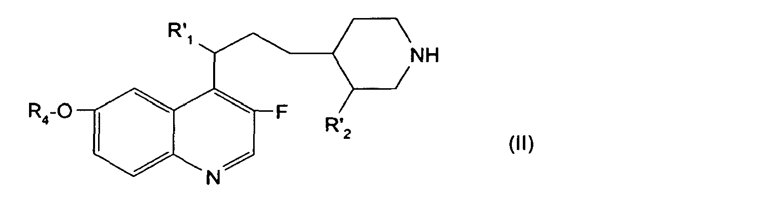 dans laquelle R₄ est défini comme précédemment, R'₁ représente un atome d'hydrogène ou un radical hydroxy et R'₂ représente un radical carboxy ou carboxyméthyle protégé, pour obtenir un dérivé de quinolyl propyl pipéridine de formule générale : 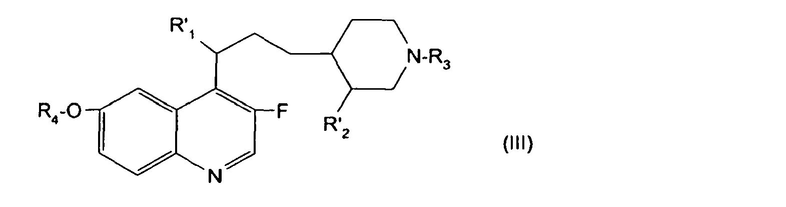 pour lequel R'₁, R'₂ et R₄ sont définis comme ci-dessus et R₃ est défini comme précédemment, puis, le cas échéant, halogénation du dérivé pour lequel R'₁ est un radical hydroxy, si l'on veut obtenir un dérivé de quinolyl propyl pipéridine pour lequel R₁ est un atome d'halogène,
ou bien, transformation du radical hydroxy représenté par R'₁en un radical oxo, puis en un radical hydroxyimino ou alcoyloxyimino, selon les méthodes connues qui n'altèrent pas le reste de la molécule, pour obtenir un dérivé de quinolyl propyl pipéridine de formule générale : 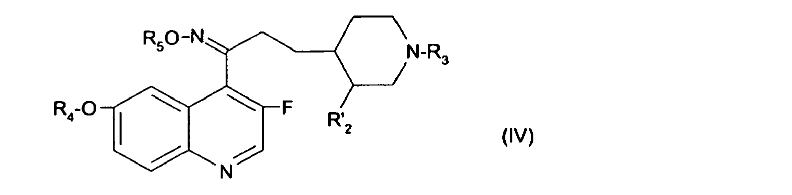 pour lequel R'₂, R₃ et R₄ sont définis comme précédemment, et R₅ est un atome d'hydrogène ou un radical alcoyle, et de la réduction du dérivé de formule générale (IV) pour lequel R₅ est un atome d'hydrogène en amine, et éventuellement de la transformation en une amine monoalcoylée ou dialcoylée, ou éventuellement de la réduction du dérivé de formule générale (IV) pour lequel R₅ est un atome d'hydrogène en hydroxylamine, ou du dérivé de formule générale (IV) pour lequel R₅ est un radical alcoyle en alcoyloxyamine, puis, le cas échéant, pour obtenir le dérivé pour lequel R₁ est alcoyl alcoyloxy amino, transforme le dérivé obtenu pour lequel R₁ est alcoyloxyamino par alcoylation,
et/ou le cas échéant réduction du radical carboxy protégé R'₂ en un radical hydroxyméthyle selon les méthodes connues qui n'altèrent pas le reste de la molécule,
puis éventuellement séparation des diastéréoisomères, séparation des formes cis et trans, élimination le cas échéant du radical protecteur d'acide, et/ou transformation du produit obtenu en un sel.

La condensation de la chaîne R₃ sur la pipéridine s'effectue avantageusement par action d'un dérivé de formule générale :

R₃-X (V)

dans laquelle R₃ est défini comme précédemment et X représente un atome d'halogène, un radical méthylsulfonyloxy, un radical trifluorométhylsulfonyloxy ou p.toluènesulfonyloxy, en opérant en milieu anhydre, de préférence inerte (azote ou argon par exemple) dans un solvant organique tel qu'un amide (diméthylformamide par exemple), une cétone (acétone par exemple) ou un nitrile (acétonitrile par exemple) en présence d'une base telle qu'une base organique azotée (par exemple triéthylamine) ou une base minérale (carbonate alcalin : carbonate de potassium par exemple) à une température comprise entre 20°C et la température de reflux du solvant.
De préférence, on fait agir un dérivé pour lequel X est un atome de brome ou d'iode.

Il est entendu que, lorsque les radicaux alcoyle représentés par R₃ portent des substituants carboxy ou amino, ces derniers sont préalablement protégés, puis libérés après la réaction. On opère selon les méthodes habituelles qui n'altèrent pas le reste de la molécule, notamment selon les méthodes décrites par T.W. Greene et P.G.M. Wuts, Protective Groups in Organic Synthesis (2^{ème} éd.), A. Wiley - Interscience Publication (1991), ou par Mc Omie, Protective Groups in Organic Chemistry, Plenum Press (1973).

Le radical carboxy ou carboxyméthyle protégé représenté par R'₂ peut être choisi parmi les esters facilement hydrolysables. A titre d'exemple peuvent être cités les esters méthylique, benzylique, tertiobutylique, ou bien les esters d'allyle ou de phénylpropyle. Eventuellement la protection du radical carboxy s'effectue simultanément à la réaction. Dans ce cas le produit de formule générale (II) mis en oeuvre porte un radical R'₂ = carboxy ou carboxyméthyle.

L'halogénation destinée à obtenir un dérivé de quinolyl propyl pidéridine pour lequel R₁ est un atome d'halogène, à partir du dérivé pour lequel R'₁ est hydroxy, peut être mise en oeuvre en présence d'un trifluorure d'aminosoufre (trifluorure de diéthylamino soufre, trifluorure de bis(2-méthoxyéthyl)amino soufre (Deoxofluor^{®}), trifluorure de morpholino soufre par exemple) ou alternativement en présence de tétrafluorure de soufre. La réaction de fluoration peut être également mise en oeuvre par action d'un agent de fluoration comme un fluorure de soufre [par exemple trifluorure de morpholino soufre, tétrafluorure de soufre (J. Org. Chem., 40, 3808 (1975)), trifluorure de diéthylamino soufre (Tetrahedron, 44, 2875 (1988)), trifluorure de bis(2-méthoxyéthyl)amino soufre (Deoxofluor^{®}). Alternativement la réaction de fluoration peut aussi s'effectuer au moyen d'un agent de fluoration comme l'hexafluoropropyl diéthylamine (JP 2 039 546) ou la N-(chloro-2 trifluoro-1,1,2 éthyl) diéthylamine. La réaction d'halogénation peut également s'effectuer au moyen d'un réactif comme un halogénure de tétra alkylammonium, de tri alkyl benzylammonium ou de tri alkyl phénylammonium ou au moyen d'un halogénure de métal alcalin additionné éventuellement d'un éther couronne.
Lorsque l'on met en oeuvre un halogénure de tétra alkylammonium, ce dernier peut être choisi, à titre d'exemple, parmi les halogénures de tétra méthylammonium, de tétra éthylammonium, de tétra propylammonium, de tétra butylammonium (tétra n-butylammonium par exemple), de tétra pentylammonium, de tétra cyclohexylammonium, de tri éthyl méthylammonium de tri butyl méthylammonium, ou de tri méthyl propylammonium.
On opère dans un solvant organique tel qu'un solvant chloré (par exemple dichlorométhane, dichloréthane, chloroforme) ou dans un éther (tétrahydrofurane, dioxanne par exemple) à une température comprise entre -78 et 40°C (de préférence entre 0 et 30°C). Il est avantageux d'opérer en milieu inerte (argon ou azote notamment).
Il est également possible d'opérer par traitement par un agent d'halogénation comme le chlorure de thionyle ou trichlorure de phosphore dans un solvant organique tel qu'un solvant chloré (dichlorométhane, chloroforme par exemple), à une température comprise entre 0°C et la température de reflux du mélange réactionnel.

La transformation du radical hydroxy en un radical oxo, s'effectue par les méthodes d'oxydation classiques, décrites dans la littérature, par exemple par oxydation de D. Swem, J.O.C., 44, 41-48 (1979) notamment en présence de chlorure d'oxalyle et de diméthylsulfoxyde, éventuellement dans un solvant comme le dichlorométhane, ou sans solvant, à une température comprise entre -60 et 20°C, puis transformation du radical oxo en un radical hydroxyimino ou alcoyloxyimino.

La transformation du radical oxo en un radical hydroxyimino ou alcoyloxyimino s'effectue par action d'hydroxylamine (chlorhydrate d'hydroxylamine par exemple) ou d'alcoyloxyamine, éventuellement sous forme de chlorhydrate, dans un solvant tel que la pyridine ou un alcool (tel que le méthanol ou l'éthanol par exemple) et en présence d'une base azotée telle que la triéthylamine ou la pyridine à une température comprise entre 0 et 60°C.

La réduction du dérivé de formule générale (IV), pour lequel R₅ est hydrogène, en amine s'effectue selon les méthodes habituelles qui n'altèrent pas le reste de la molécule, notamment par action d'un agent réducteur comme par exemple un hydrure (borohydrure alcalin : borohydrure de sodium ou de potassium par exemple ou hydrure d'aluminium et de lithium) en présence ou non d'oxyde de molybdène, en opérant de préférence sous atmosphère inerte (azote ou argon par exemple), dans un solvant organique comme un alcool (méthanol, éthanol, isopropanol par exemple) ou un solvant chloré (par exemple dichorométhane) à une température comprise entre -10 et 40°C.

La réduction du dérivé de formule générale (IV) en hydroxylamine ou en alcoyloxyamine s'effectue notamment en présence d'un acide organique (acide carboxylique comme par exemple l'acide acétique), par action d'un agent réducteur comme par exemple un hydrure choisi parmi le triacétoxy-borohydrure de sodium (éventuellement préparé in situ) ou le cyanoborohydrure de sodium, de préférence sous atmosphère inerte (azote ou argon par exemple), dans un solvant organique comme un alcool (méthanol, éthanol, isopropanol par exemple) ou un solvant chloré (par exemple dichorométhane) à une température comprise entre -30 et +40°C.

La transformation du radical amino représenté par R₁ en un radical alcoylamino ou dialcoylamino s'effectue selon les méthodes habituelles, notamment par action d'un halogénure d'alcoyle, éventuellement en milieu basique en présence d'une base azotée comme une trialcoylamine (triéthylamine, diisopropyl éthyl amine ...), la pyridine, ou en présence d'un hydrure de métal alcalin (hydrure de sodium), dans un solvant inerte comme un amide (diméthylformamide par exemple) ou un oxyde (diméthylsulfoxyde par exemple), à une température comprise entre 20°C et la température de reflux du mélange réactionnel.

La transformation du radical alcoyloxyamino représenté par R₁ en un radical alcoyl alcoyloxy amino s'effectue selon la méthode décrite ci-dessus pour l'alcoylation de l'aminé.

L'élimination, le cas échéant, du radical protecteur d'acide pour obtenir un dérivé de quinolyl propyl pipéridine pour lequel R₂ est un radical carboxy ou carboxyméthyle, s'effectue selon les méthodes habituelles, notamment par hydrolyse acide ou saponification de l'ester R'₂. Notamment on fait agir la soude en milieu hydroorganique, par exemple dans un alcool comme le méthanol ou un éther comme le dioxanne, à une température comprise entre 20°C et la température de reflux du mélange réactionnel. On peut également mettre en oeuvre l'hydrolyse en milieu chlorhydrique aqueux à une température comprise entre 20 et 100°C.

Le cas échéant, le dérivé de formule générale (1) pour lequel R₂ est hydroxyméthyle peut être préparé à partir du dérivé pour lequel R'₂ est carboxy protégé. Notamment, on opère par réduction du produit protégé sous forme d'un ester R'₂, selon les méthodes habituelles qui n'altèrent pas le reste de la molécule, en particulier par action d'un hydrure (hydrure d'aluminium et de lithium ou hydrure de diisobutyl aluminium par exemple) dans un solvant tel qu'un éther (tétrahydrofurane par exemple) à une température comprise entre 20 et 60°C.

Le dérivé de quinolyl propyl pipéridine de formule générale (II) pour lequel R'₂ représente un radical carboxyméthyle protégé, et R'₁ est un atome d'hydrogène, peut être préparé par hydrogénation sélective du dérivé de quinolyl propyl pipéridine de formule générale : 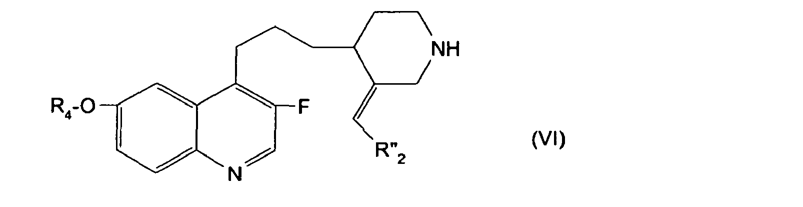 dans laquelle R₄ est défini comme précédemment et R"₂ est le radical carboxy protégé correspondant à R'₂, et dont la fonction amine de la pipéridine est préalablement protégée, sous une pression de 1 à 100 bars et à une température comprise entre 20 et 80°C, dans un solvant comme notamment un alcool (éthanol par exemple) ou un amide (diméthylformamide par exemple) en présence d'un catalyseur, par exemple le palladium sur charbon ou le palladium sur sulfate de baryum.
La protection de l'amino de la pipéridine s'effectue selon les méthodes habituelles qui n'altèrent pas le reste de la molécule et compatibles avec la réaction ; notamment selon les références citées ci-avant. Le radical protecteur est plus particulièrement le radical benzyloxycarbonyle. Dans ce cas la réaction d'hydrogénation conduit directement à la déprotection de l'amine.

Le dérivé de quinolyl propyl pipéridine de formule générale (VI) peut être préparé par condensation d'un dérivé de quinoléine de formule générale : 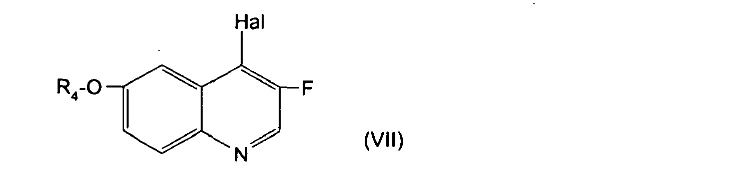 dans laquelle R₄ est défini comme précédemment et Hal représente un atome d'iode ou de brome, sur un dérivé de la pipéridine de formule générale : 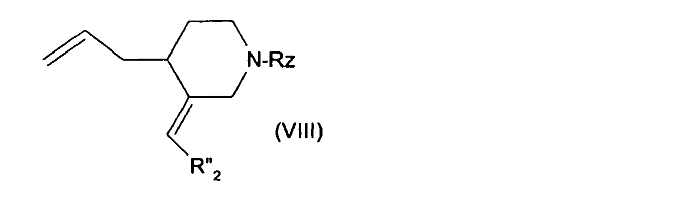 dans laquelle R"₂ est défini comme ci-dessus et R_{z} représente un radical protecteur d'amino.

La réaction s'effectue par action successive d'un organoborane (9-borabicyclo[3,3,1]nonane par exemple) dans un solvant tel qu'un éther (tétrahydrofuranne, dioxanne par exemple) à une température comprise entre -20 et 20°C, puis de l'addition du dérivé de quinoléine de formule générale (VII), par analogie avec les méthodes décrites par Suzuki et al., Pure and Appl. Chem., 57, 1749 (1985). La réaction s'effectue généralement en présence d'un sel de palladium (chlorure de palladium diphénylphosphinoferrocène par exemple) et d'une base comme le phosphate de potassium à une température comprise entre 20°C et la température de reflux du solvant.

Le dérivé de la pipéridine de formule générale (VIII) peut être préparé par réaction de Wittig, par condensation d'un ylure de phosphore sur un dérivé de pipéridine de formule générale : 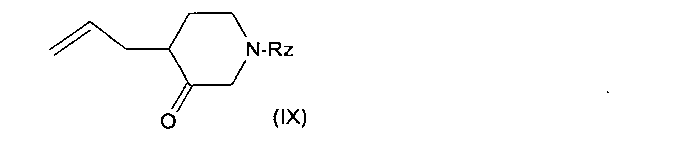 dans laquelle Rz est défini comme ci-dessus.

On opère avantageusement au moyen de (triphénylphosphoranylidène) acétate de méthyle, dans un solvant comme par exemple le toluène, à une température comprise entre 20 et 110°C.

Le dérivé d'oxo-3 pipéridine de formule générale (IX) peut être préparé selon ou par analogie avec la méthode décrite par Y. Takeuchi et coll., Synthesis, 10, 1814 (1999).

Le dérivé de quinolyl propyl pipéridine de formule générale (II), pour lequel R'₂ est un radical carboxy et R'₁ est un atome d'hydrogène peut être préparé à partir du dérivé correspondant pour lequel R'₂ est carboxyméthyle protégé, par réduction de ce radical en alcool, transformation en un dérivé p.toluènesulfonyloxy, puis transformation de ce dérivé en dérivé vinylique par réaction d'élimination suivie de l'oxydation du dérivé obtenu.

Selon une autre alternative le dérivé de quinolyl propyl pipéridine de formule générale (II), pour lequel R'₂ est un radical carboxy et R'₁ est un atome d'hydrogène peut être préparé par condensation d'un dérivé de la quinoléine de formule générale (VIII) tel que défini précédemment, sur un dérivé de pipéridine de formule générale : 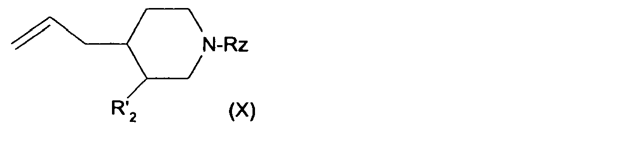 dans laquelle Rz est défini comme précédemment et R'₂ représente un radical carboxy protégé tel que défini précédemment, puis élimine le radical Rz protecteur d'amino.

La réaction s'effectue dans des conditions analogues aux conditions décrites pour la réaction du dérivé de quinoléine de formule générale (VII) et du dérivé de la pipéridine de formule générale (VIII).
L'élimination du radical Rz s'effectue selon les méthodes connues et citées ci avant, citées dans les exemples, ou décrites par T.W. Greene et P.G.M. Wuts, Protective Groups in Organic Synthesis (2^{ème} éd.), A. Wiley - Interscience Publication (1991), ou par Mc Omie, Protective Groups in Organic Chemistry, Plenum Press (1973).

Le produit de formule générale (X) peut être préparé selon ou par analogie avec la méthode décrite ci-après dans les exemples.

La réduction en alcool de l'acide protégé sous forme d'un radical R'₂ en position -3 de la pipéridine, en un radical hydroxyéthyle s'effectue selon les méthodes habituelles qui n'altèrent pas le reste de la molécule, notamment on opère par action d'un hydrure (hydrure d'aluminium et de lithium ou hydrure de diisobutyl aluminium par exemple) dans un solvant tel qu'un éther (tétrahydrofurane par exemple) à une température comprise entre 20 et 60°C.
La transformation du dérivé de quinolyl propyl pipéridine pour lequel R'₂ est hydroxyéthyle en un dérivé p.toluènesulfonyloxyéthyle s'effectue notamment selon la méthode décrite par L.F. Fieser et M. Fieser, Reagents for Organic Synthesis, vol 1, 1179 (1967), à partir du chlorure de p.toluènesulfonyle en présence d'une base comme une amine tertiaire (par exemple la triéthylamine) ou aromatique (par exemple la pyridine), dans un solvant halogéné (dichlorométhane par exemple) ou sans solvant, à une température comprise entre 0 et 50°C.
La transformation du dérivé p.toluènesulfonyloxyéthyle en dérivé vinylique s'effectue par réaction d'élimination, notamment selon la méthode décrite par A. Sharma et coll., Org. Prep Proced. Int., 25(3), 330-333 (1993), en présence d'une base comme par exemple le t.butylate de potassium dans un solvant tel que le diméthylsulfoxyde par exemple, à une température comprise entre 20 et 100°C.

La transformation du dérivé vinylique en un dérivé pour lequel R'₂ est carboxy s'effectue par les méthodes d'oxydation décrites dans la littérature, notamment par le méta periodate de sodium en présence d'hydrate de trichlorure de ruthénium, dans un mélange de solvants comme par exemple le mélange eau/acétonitrile, à une température comprise entre 20 et 60°C.

Le dérivé de quinolyl propyl pipéridine de formule générale (II), pour lequel R'₁ est un radical hydroxy peut être préparé par oxydation en milieu basique du dérivé correspondant pour lequel R'₁ est un atome d'hydrogène. L'oxydation s'effectue par action de l'oxygène, de préférence au sein d'un solvant inerte tel que le diméthylsulfoxyde en présence de tert-butanol et d'une base telle le tert-butylate de potassium ou de sodium à une température comprise entre 0 et 100°C.

Les dérivés de la quinoléine de formule générale (VII) pour lesquels Hal est un atome d'iode, peuvent être préparés par analogie avec les travaux de E. Arzel et al., Tetrahedron, 55, 12149-12156 (1999) à partir de 3-fluoro-6-méthoxyquinoléine, par action successive d'une base puis de l'iode. On utilise par exemple le diisopropylamidure de lithium dans un solvant tel qu'un éther (tétrahydrofuranne) à une température comprise entre -80 et 20°C. La 3-fluoro-6-méthoxyquinoléine peut être obtenue par pyrolyse du 3-tétrafluoroborate ou du 3-hexafluorophosphate de diazonium de 6-méthoxyquinoléine selon la réaction de Balz-Schieman, Org. Synth., Coll 5, 133 (1973), à une température comprise entre 100 et 240°C. Le 3-tétrafluoroborate de diazonium de 6-méthoxyquinoléine ou le 3-hexafluorophophate de diazonium de 6-méthoxyquinoléine peuvent être obtenus à partir de la 3-amino-6-méthoxyquinoléine par action d'un nitrite alcalin (nitrite de sodium par exemple) en milieu acide (acide tétrafluoroborique ou acide hexafluorophophorique) dans un solvant tel que l'eau à une température comprise entre -10 et +20°C, par analogie avec les travaux de A. Roe et al., J. Am. Chem. Soc., 71, 1785-86 (1949) ou par action d'un nitrite d'alcoyle (comme par exemple le nitrite d'isoamyle) et du complexe de trifluoroborate diéthyléther dans un solvant tel qu'un éther (tétrahydrofuranne par exemple) à une température comprise entre -10 et +10°C. La 3-amino-6-méthoxyquinoléine est préparée comme décrit par N. Heindel, J. Med. Chem., 13, 760 (1970). Le dérivé de la quinoléine de formule générale (VII) pour lequel Hal est un atome de brome, peuvt être également préparés par analogie avec cette méthode.

Les intermédiaires des dérivés de quinolyl propyl pipéridine pour lesquels R₄ représente alcényl-CH₂-, alcynyl-CH₂-, cycloalcoyle ou cycloalcoyl alcoyle peuvent être obtenus par analogie avec la préparation des intermédiaires pour lesquels R₄ est alcoyle, par action du dérivé halogéné correspondant sur le dérivé de quinoléine hydroxylé en position -6.

Il est entendu que les dérivés de formule générale (I), (II), (III), (IV) ou leurs intermédiaires de départ peuvent exister sous forme cis ou trans au niveau des substituants en position -3 et -4 de la pipéridine. Les dérivés de configuration trans peuvent être obtenus à partir des dérivés de configuration cis selon ou par analogie avec la méthode décrite dans la demande internationale WO 99/37635.

Les dérivés de quinolyl propyl pipéridine de formule générale (I) peuvent être purifiés le cas échéant par des méthodes physiques telles que la cristallisation ou la chromatographie.

Par ailleurs il est entendu que lorsque R₁ est autre que l'atome d'hydrogène, il existe des formes diastéréoisomères et que les formes diastéréoisomères et leurs mélanges entrent aussi dans le cadre de la présente invention. Ces derniers peuvent être notamment séparés par chromatographie sur silice ou par Chromatographie Liquide Haute Performance (CLHP). De même, les dérivés cis et trans peuvent être séparés par chromatographie sur silice ou par Chromatographie Liquide Haute Performance (CLHP).

Les dérivés de quinolyl propyl pipéridine de formule générale (I) peuvent être transformés en sels d'addition avec les acides, par les méthodes connues. Il est entendu que ces sels entrent aussi dans le cadre de la présente invention.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, phénylsulfonates, p.toluènesulfonates, iséthionates, naphtylsulfonates ou camphorsulfonates, ou avec des dérivés de substitution de ces composés).

Certains des dérivés de quinolyl propyl pipéridine de formule générale (I) portant un radical carboxy peuvent être transformés à l'état de sels métalliques ou en sels d'addition avec les bases azotées selon les méthodes connues en soi. Ces sels entrent également dans le cadre de la présente invention. Les sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhydrylamine, quinine, choline, arginine, lysine, leucine, dibenzylamine).

Les dérivés de quinolyl propyl pipéridine selon l'invention sont des agents antibactériens particulièrement intéressants.

In vitro, sur germes gram positifs les dérivés de quinolyl propyl pipéridine selon l'invention se sont montrés actifs à des concentrations comprises entre 0,03 et 4 µg/ml sur *Staphylococcus aureus* AS5155 résistante à la méticilline, également à des concentrations comprises entre 0,06 et 8 µg/ml sur *Streptococcus pneumoniae* 6254-01 et à des concentrations comprises entre 0,06 et 64 µg/ml sur *Enterococcus faecium* H983401 et sur germes gram négatifs, ils se sont montrés actifs à des concentrations comprises entre 0,12 et 32 µg/ml sur *Moraxella catharrhalis* IPA152 ; in vivo, ils se sont montrés actifs sur les infections expérimentales de la souris à *Staphylococcus aureus* IP8203 à des doses comprises entre 12 et 150 mg/kg par voie sous cutanée (DC₅₀) et pour certains d'entre eux à des doses comprises entre 26 et 150 mg/kg par voie orale.

Enfin, les produits selon l'invention sont particulièrement intéressants du fait de leur faible toxicité. Aucun des produits n'a manifesté de toxicité à la dose de 100 mg/kg par voie sous cutanée chez la souris (2 administrations).

Parmi les produits selon l'invention, peuvent être cités particulièrement les dérivés de quinolyl propyl quinoléine décrits ci-après :
- Acide (3RS,4RS) ou (3SR,4RS)-4-[3-(3-fluoro-6-méthoxyquinolin-4-yl)propyl]-1-(2-phénylthioéthyl)pipéridine-3-carboxylique
- Acide (3RS,4RS) ou (3SR,4RS)-4-[3-(3-fluoro-6-méthoxyquinolin-4-yl)propyl]-1-[2-(3-fluorophénylthio)éthyl]pipéridine-3-carboxylique
- Acide (3RS,4RS) ou (3SR,4RS)-4-[3-(3-fluoro-6-méthoxyquinolin-4-yl)propyl]-1-[2-(2,5-difluorophénylthio)éthyl]pipéridine-3-carboxylique
- Acide (3RS,4RS) ou (3SR,4RS)-4-[3-(3-fluoro-6-méthoxyquinolin-4-yl)propyl]-1-[2-(3,5-difluorophénylthio)éthyl]pipéridine-3-carboxylique
- Acide (3RS,4RS) ou (3SR,4RS)-4-[3-(3-fluoro-6-méthoxyquinolin-4-yl)propyl]-1-[2-(2,3,5-trifluorophénylthio)éthyl]pipéridine-3-carboxylique
- Acide (3RS,4RS) ou (3SR,4RS)-4-[3-(3-fluoro-6-méthoxyquinolin-4-yl)propyl]-1-[3-(n-propylthio)propyl]pipéridine-3-carboxylique
- Acide (3RS,4RS) ou (3SR,4RS)-4-[3-(3-fluoro-6-méthoxyquinolin-4-yl)propyl]-1-[2-(n-butylthio)éthyl]pipéridine-3-carboxylique
- Acide (3RS,4RS) ou (3SR,4RS)-4-[3-(3-fluoro-6-méthoxyquinolin-4-yl)propyl]-1-[2-(cyclopropylthio)éthyl]pipéridine-3-carboxylique
- Acide (3RS,4RS) ou (3SR,4RS)-4-[3-(3-fluoro-6-méthoxyquinolin-4-yl)propyl]-1-[2-(cyclobutylthio)éthyl]pipéridine-3-carboxylique
- Acide (3RS,4RS) ou (3SR,4RS)-4-[3-(3-fluoro-6-méthoxyquinolin-4-yl)propy)]-1-[2-(cyclopentylthio)éthyl]pipéridine-3-carboxylique
- Acide (3RS,4RS) ou (3SR,4RS)-4-[3-(3-fluoro-6-méthoxyquinolin-4-yl)propyl]-1-[2-(cyclohexylthio)éthyl]pipéridine-3-carboxylique
- Acide (3RS,4RS) ou (3SR,4RS)-4-[3-(3-fluoro-6-méthoxyquinolin-4-yl)propyl]-1-[2-(thien-2-yl)thioéthyl]pipéridine-3-carboxylique
- Acide (3RS,4RS) ou (3SR,4RS)-4-[3-(3-fluoro-6-méthoxyquinolin-4-yl)propyl]-1-[2-(5-fluorothien-2-yl)thioéthyl]pipéridine-3-carboxylique
- Acide (3RS,4RS) ou (3SR,4RS)-4-[3-(3-fluoro-6-méthoxyquinolin-4-yl)propyl]-1-[2-(thien-3-yl)thioéthyl]pipéridine-3-carboxylique
- Acide (3RS,4RS) ou (3SR,4RS)-4-[3-(3-fluoro-6-méthoxyquinolin-4-yl)propyl]-1-[2-(1,3-thiazol-2-yl)thioéthyl]pipéridine-3-carboxylique
- Acide (3RS,4RS) ou (3SR,4RS)-4-[3-(3-fluoro-6-méthoxyquinolin-4-yl)propyl]-1-[2-(pyridin-2-yl)thioéthyl]pipéridine-3-carboxylique
- Acide (3RS,4RS) ou (3SR,4RS)-4-[3-(3-fluoro-6-méthoxyquinolin-40-yl)propyl]-1-[2-(3-fluoro-pyridin-2-yl)thioéthyl]pipéridine-3-carboxylique
- Acide (3RS,4RS) ou (3SR,4RS)4-[3-(R,S)-hydroxy-3-(3-fluoro-6-méthoxyquinolin-4-yl)propyl]-1-(2-phénylthioéthyl)pipéridine-3-carboxylique
- Acide (3RS,4RS) ou (3SR,4RS)-4-[3-(R,S)-hydroxy-3-(3-fluoro-6-méthoxyquinolin-4-yl)propyl]-1-[2-(3-fluorophénylthio)éthyl]pipéridine-3-carboxylique
- Acide (3RS,4RS) ou (3SR,4RS)-4-[3-(R,S)-hydroxy-3-(3-fluoro-6-méthoxyquinolin-4-yl)propyl]-1-[2-(2,5-difluorophénylthio)éthyl]pipéridine-3-carboxylique
- Acide (3RS,4RS) ou (3SR,4RS)-4-[3-(R,S)-hydroxy-3-(3-fluoro-6-méthoxyquinolin-4-yl)propyl]-1-[2-(3,5-difluorophénylthio)éthyl]pipéridine-3-carboxylique
- Acide (3RS,4RS) ou (3SR,4RS)-4-[3-(R,S)-hydroxy-3-(3-fluoro-6-méthoxyquinolin-4-yl)propyl]-1-[2-(2,3,5-trifluorophénylthio)éthyl]pipéridine-3-carboxylique
- Acide (3RS,4RS) ou (3SR,4RS)-4-[3-(R,S)-hydroxy-3-(3-fluoro-6-méthoxyquinolin-4-yl)propyl]-1-[3(n-propylthio)éthyl]pipéridine-3-carboxylique
- Acide (3RS,4RS) ou (3SR,4RS)-4-[3-(R,S)-hydroxy-3-(3-fluoro-6-méthoxyquinolin-4-yl)propyl]-1-[2-(n-butylthio)éthyl]pipéridine-3-carboxylique
- Acide (3RS,4RS) ou (3SR,4RS)-4-[3-(R,S)hydroxy-3-(3-fluoro-6-méthoxyquinolin-4-yl)propyl]-1-[2-(cyclopropylthio)éthyl]pipéridine-3-carboxylique
- Acide (3RS,4RS) ou (3SR,4RS)-4-[3-(R,S)-hydroxy-3-(3-fluoro-6-méthoxyquinolin-4-yl)propyl]-1-[2-(cyclobutylthio)éthyl]pipéridine-3-carboxylique
- Acide (3RS,4RS) ou (3SR,4RS)-4-[3-(R,S)-hydroxy-3-(3-fluoro-6-méthoxyquinolin-4-yl)propyl]-1-[2-(cyclopentylthio)éthyl]pipéridine-3-carboxylique
- Acide (3RS,4RS) ou (3SR,4RS)-4-[3-(R,S)-hydroxy-3-(3-fluoro-6-méthoxyquinolin-4-yl)propyl]-1-[2-(cyclohexylthio)éthyl]pipéridine-3-carboxylique
- Acide (3RS,4RS) ou (3SR,4RS)-4-[3-(R,S)-hydroxy-3-(3-fluoro-6-méthoxyquinolin-4-yl)propyl]-1-[2-(5-fluoro-thien-2-yl)thioéthyl]pipéridine-3-carboxylique
- Acide (3RS,4RS) ou (3SR,4RS)-4-[3-(R,S)-hydroxy-3-(3-fluoro-6-méthoxyquinolin-4-yl)propyl]-1-[2-(thien-3-yl)thioéthyl]pipéridine-3-carboxylique
- Acide (3RS,4RS) ou (3SR,4RS)-4-[3-(R,S)-hydroxy-3-(3-fluoro-6-méthoxyquinolin-4-yl)propyl]-1-[2-(1,3-thiazol-2-yl)thioéthyl]pipéridine-3-carboxylique
- Acide (3RS,4RS) ou (3SR,4RS)-4-[3-(R,S)-hydroxy-3-(3-fluoro-6-méthoxyquinolin-4-yl)propyl]-1-[2-(3-fluoro-pyridin-2-yl)thioéthyl]pipéridine-3-carboxylique
- Acide (3RS,4RS) ou (3SR,4RS)-4-[3-(R,S)-hydroxy-3-(3-fluoro-6-méthoxyquinolin-4-yl)propyl]-l- [2 -(pyridin- 2- yl)thioéthyl]pipéridine- 3-carboxylique
- Acide (3RS,4RS) ou (3SR,4RS)-4-[3-(R,S)-fluoro-3-(3-fluoro-6-méthoxyquinolin-4-yl)propyl]-1-[2-(2,5-difluorophénylthio)éthyl]pipéridine-3-carboxylique
- Acide (3RS,4RS) ou (3SR,4RS)-4-[3-(R,S)-fluoro-3-(3-fluoro-6-méthoxyquinolin-4-yl)propyl]-1-[2-(3,5-difluorophénylthio)éthyl]pipéridine-3-carboxylique
- Acide (3RS,4RS) ou (3SR,4RS)-4-[3-(RS)-fluoro-3-(3-fluoro-6-méthoxyquinolin-4-yl)propyl]-1-[2-(cyclopentylthio)éthyl]pipéridine-3-carboxylique
- Acide (3RS,4RS) ou (3SR,4RS)-4-[3-(R,S)-fluoro-3-(3-fluoro)-6-méthoxyquinolin-4-yl)propyl]-1-[2-(cyclohexylthio)éthyl]pipéridine-3-carboxylique
- Acide (3RS,4RS) ou (3SR,4RS)-4-[3-(R,S)-fluoro-3-(3-fluoro-6-méthoxyquinolin-4-yl)propyl]-1-[2-(thien-2-yl)thioéthyl]pipéridine-3-carboxylique
- Acide (3RS,4RS) ou (3SR,4RS)-4-[3-(R,S)-fluoro-3-(3-fluoro-6-méthoxyquinolin-4-yl)propyl]-1-[2-(1,3-thiazol-2-yl)thioéthyl]pipéridine-3-carboxylique
- Acide (3RS,4RS) ou (3SR,4RS)-4-[3-(R,S)-fluoro-3-(3-fluoro-6-méthoxyquinolin-4-yl)propyl]-1-[2-(pyridin-2-yl)thioéthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-(2-phénylthioéthyl)pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(3-fluorophénylthio)éthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(2,5-difluorophénylthio)éthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(3,5-difluorophénylthio)éthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(2,3,5-trifluorophénylthio)éthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[3-(n-propylthio)propyl]pipéridine-3-carboxylique
- Acide (3FLS,4RS) ou (3SR,4RS)-4-[3-(R,S)-amino-3-(3-fluoro)-6-méthoxyquinolin-4-yl)propyl]-1-[2-(n-butylthio)éthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(cyclopropylthio)éthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(cyclobutylthio)éthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(cyclopentylthio)éthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(cyclohexylthio)éthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(thien-2-yl)thioéthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(5-fluoro-thien-2-yl)thioéthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(thien-3-yl)thioéthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(1,3-thiazol-2-yl)thioéthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(pyridin-2-yl)thioéthyl]pipéridine-3-carboxylique
- Acide (3RS,4RS) ou (3SR,4RS)-4-[3-(R,S)-amino-3-(3-fluoro-6-méthoxyquinolin-4-yl)propyl]-1-[2-(pyridin-3-yl)thioéthyl]pipéridine-3-carboxylique
- Acide (3RS,4RS) ou (3SR,4RS)-4-[3-(3-fluoro-6-méthoxyquinolin-4-yl)propyl]-1-(2-phénylthioéthyl)pipéridine-3-acétique
- Acide (3RS,4RS) ou (3SR,4RS)-4-[3-(3-fluoro-6-méthoxyquinolin-4-yl)propyl]-1-[2-(3-fluorophénylthio)éthyl]pipéridine-3-acétique
- Acide (3RS,4RS) ou (3SR,4RS)-4-[3-(3-fluoro-6-méthoxyquinolin-4-yl)propyl]-1-[2-(2,5-difluorophénylthio)éthyl]pipéridine-3-acétique
- Acide (3RS,4RS) ou (3SR,4RS)-4-[3-(3-fluoro-6-méthoxyquinolin-4-yl)propyl]-1-[2-(3,5-difluorophénylthio)éthyl]pipéridine-3-acétique
- Acide (3RS,4RS) ou (3SR,4RS)-4-[3-(3-fluoro-6-méthoxyquinolin-4-yl)propyl]-1-[2-(2,3,5-trifluorophénylthio)éthyl]pipéridine-3-acétique
- Acide (3RS,4RS) ou (3SR,4RS)-4-[3-(3-fluoro-6-méthoxyquinolin-4-yl)propyl]-I-[3-(n-propylthio)propyl]pipéridine-3-acétique
- Acide (3RS,4RS) ou (3SR,4RS)-4-[3-(3-fluoro-6-méthoxyquinolin-4-yl)propyl]-1-[2-(n-butylthio)éthyl]pipéridine-3-acétique
- Acide (3RS,4RS) ou (3SR,4RS)-4-[3-(3-fluoro-6-méthoxyquinolin-4-yl)propyl]-1-[2-(cyclopropylthio)éthyl]pipéridine-3-acétique
- Acide (3RS,4RS) ou (3SR,4RS)-4-[3-(3-fluoro-6-méthoxyquinolin-4-yl)propyl]-I-[2-(cyclobutylthio)éthyl]pipéridine-3-acétique
- Acide (3RS,4RS) ou (3SR,4RS)-4-[3-(3-fluoro-6-méthoxyquinolin-4-yl)propyl]-1-[2-(cyclopentylthio)éthyl]pipéridine-3-acétique
- Acide (3RS,4RS) ou (3SR,4RS)-4-[3-(3-fluoro-6-méthoxyquinolin-4-yl)propyl]-1-[2-(cyclohexylthio)éthyl]pipéridine-3-acétique
- Acide (3RS,4RS) ou (3SR,4RS)-4-[3-(3-fluoro-6-méthoxyquinolin-4-yl)propyl]-1-[2-(5-fluoro-thien-2-yl)thioéthyl]pipéridine-3-acétique
- Acide (3RS,4RS) ou (3SR,4RS)-4-[3-(3-fluoro-6-méthoxyquinolin-4-yl)propyl]-1-[2-(thien-3-yl)thioéthyl]pipéridine-3-acétique
- Acide (3RS,4RS) ou (3SR,4RS)-4-[3-(3-fluoro-6-méthoxyquinolin-4-yl)propyl]-1-[2-(1,3-thiazol-2-yl)thioéthyl]pipéridine-3-acétique
- Acide (3RS,4RS) ou (3SR,4RS)-4-[3-(3-fluoro-6-méthoxyquinolin-4-yl)propyl]-1-[2-(pyridin-2-yl)thioéthyl]pipéridine-3-acétique
- Acide (3RS,4RS) ou (3SR,4RS)-4-[3-(3-fluoro-6-méthoxyquinolin-4-yl)propyl]-1-[2-(3-fluoro-pyridin-2-yl)thioéthyl]pipéridine-3-acétique
- Acide (3RS,4RS) ou (3SR,4RS)-4-[3-(R,S)-hydroxy-3-(3-fluoro-6-méthoxyquinolin-4-yl)propyl]-1-(2-phénylthioéthyl)pipéridine-3-acétique
- Acide (3RS,4RS) ou (3SR,4RS)-4-[3-(R,S)-hydroxy-3-(3-fluoro-6-méthoxyquinolin-4-yl)propyl]-1-[2-(3-fluorophénylthio)éthyl]pipéridine-3-acétique
- Acide (3RS,4RS) ou (3SR,4RS)-4-[3-(R,S)-hydroxy-3-(3-fluoro)-6-méthoxyquinolin-4-yl)propyl]-1-[2-(3,5-difluorophénylthio)éthyl]pipéridine-3-acétique
- Acide (3RS,4RS) ou (3SR,4RS)-4-[3-(R,S)-hydroxy-3-(3-fluoro-6-méthoxyquinolin-4-yl)propyl]-1-[2-(2,3,5-trifluorophénylthio)éthyl]pipéridine-3-acétique
- Acide (3RS,4RS) ou (3SR,4RS)-4-[3-(R,S)-hyaroxy-3-(3-auoro-6-méthoxyquinolin-4-yl)propyl]-1-[3-(n-propylthio)propyl]pipéridine-3-acétique
- Acide (3RS,4RS) ou (3SR,4RS)-4-[3-(R,S)-hydroxy-3-(3-fluoro-6-méthoxyquinolin-4-yl)propyl]-1-[2-(n-butylthio)éthyl]pipéridine-3-acétique
- Acide (3RS,4RS) ou (3SR,4RS)-4-[3-(R,S)-hydroxy-3-(3-fluoro-6-méthoxyquinolin-4-yl)propyl]-1-[2-(cyclopropylthio)éthyl]pipéridine-3-acétique
- Acide (3RS,4RS) ou (3SR,4RS)-4-[3-(R,S)-hydroxy-3-(3-fluoro-6-méthoxyquinolin-4-yl)propyl]-1-[2-(cyclobutylthio)éthyl]pipéridine-3-acétique
- Acide (3RS,4RS) ou (3SR,4RS)-4-[3-(R,S)-hydroxy-3-(3-fluoro-6-méthoxyquinolin-4-yl)propyl]-1-[2-(cyclohexylthio)ethyl]pipéridine-3-acétique
- Acide (3RS,4RS) ou (3SK4RS)-4-[3-(RS)-hydroxy-3-(3-fluoro-6-méthoxyquinolin-4-yl)propyl]-1-[2-(5-fluoro-thien-2-yl)thioéthyl]pipéridine-3-acétique
- Acide (3RS,4RS) ou (3SR,4RS)-4-[3-(R,S)-hydroxy-3-(3-fluoro-6-méthoxyquinolin-4-yl)propyl]-1-[2-(thien-3-yl)thioéthyl]pipéridine-3-acétique
- Acide (3RS,4RS) ou (3SR,4RS)-4-[3-(R,S)-hydroxy-3-(3-fluoro-6-méthoxyquinolin-4-yl)propyl]-1-[2-(1,3-thiazol-2-yl)thioéthyl]pipéridine-3-acétique
- Acide (3RS,4RS) ou (3SR,4RS)-4-[3-(R,S)hydroxy-3-(3-fluoro-6-méthoxyquinolin-4-yl)propyl]-1-[2-(pyridin-2-yl)thioéthyl]pipéridine-3-acétique
- Acide (3RS,4RS) ou (3SR,4RS)-4-[3-(K,S)-hydroxy-3-(3-fluoro-6-méthoxyquinolin-4-yl)propyl]-1-[3-(thien-3-yl)-prop-2-ynyl]-pipéridine-3-acétique
- Acide (3RS,4RS) ou (3SR,4RS)-4-[3-(R,S)-fluoro-3-(3-fluoro-6-méthoxyquinolin-4-yl)propyl]-I-[2-(2,5-difluorophénylthio)éthyl]pipéridine-3-acétique
- Acide (3RS,4RS) ou (3SR,4RS)-4-[3-(R,S)-fluoro-3-(3-fluoro-6-méthoxyquinolin-4-yl)propyl]-1-[2-(3,5-difluorophénylthio)éthyl]pipéridine-3-acétique
- Acide (3RS,4RS) ou (3SR,4RS)-4-[3-(R,S)-fluoro-3-(3-fluoro-6-méthoxyquinolin-4-yl)propyl]-1-[2-(cyclopentylthio)éthyl]pipéridine-3-acétique
- Acide (3RS,4RS) ou (3SR,4RS)-4-[3-(R,S)-fluoro-3-(3-fluoro-6-méthoxyquinolin-4-yl)propyl]-1-[2-(cyclohexylthio)éthyl]pipéridine-3-acétique
- Acide (3RS,4RS) ou (3SR,4RS)-4-[3-(R,S)-fluoro-3-(3-fluoro-6-méthoxyquinolin-4-yl)propyl]-1-[2-(thien-2-yl)thioéthyl]pipéridine-3-acétique
- Acide (3RS,4RS) ou (3SR,4RS)-4-[3-(R,S)-fluoro-3-(3-fluoro-6-méthoxyquinolin-4-yl)propyl]-1-[2-(1,3-thiazol-2-yl)thioéthyl]pipéridine-3-acétique
- Acide (3RS,4RS) ou (3SR,4RS)-4-[3-(R,S)-fluoro-3-(3-fluoro-6-méthoxyquinolin-4-yl)propyl]-1-[2-(pyridin-2-yl)thioéthyl]pipéridine-3-acétique
- Acide (3RS,4RS) ou (3SR,4RS)-4-[3-(R,S)-amino-3-(3-fluoro-6-méthoxyquinolin-4-yl)propyl]-1-(2-phénylthioéthyl)pipéridine-3-acétique
- Acide (3RS,4RS) ou (3SR,4RS)-4-[3-(R,S)-amino-3-(3-fluoro-6-méthoxyquinolin-4-yl)propyl]-1-[2-(3-fluorophénylthio)éthyl]pipéridine-3-acétique
- Acide (3RS,4RS) ou (3SR,4RS)-4-[3-(R,S)-amino-3-(3-fluoro-6-méthoxyquinolin-4-yl)propyl]-1-[2-(3,5-difluorophénylthio)éthyl]pipéridine-3-acétique
- Acide (3RS,4RS) ou (3SR,4RS)-4-[3-(R,S)-amino-3-(3-fluoro-6-méthoxyquinolin-4-yl)propyl]-1-[2-(2,3,5-trifluorophénylthio)éthyl]pipéridine-3-acétique
- Acide (3RS,4RS) ou (3SR,4RS)-4-[3-(R,S)-amino-3-(3-fluoro-6-méthoxyquinolin-4-yl)propyl]-1-[3-(n-propylthio)propyl]pipéridine-3-acétique
- Acide (3RS,4RS) ou (3SR,4RS)-4-[3-(R,S)-amino-3-(3-fluoro-6-méthoxyquinolin-4-yl)propyl]-1-[2-(n-butylthio)éthyl]pipéridine-3-acétique
- Acide (3RS,4RS) ou (3SR,4RS)-4-[3-(R,S)-amino-3-(3-fluoro-6-méthoxyquinolin-4-yl)propyl]-1-[2-(cyclopropylthio)éthyl]pipéridine-3-acétique
- Acide (3RS,4RS) ou (3SR,4RS)-4-[3-(R,S)-amino-3-(3-fluoro-6-méthoxyquinolin-4-yl)propyl]-1-[2-(cyclobutylthio)éthyl]pipéridine-3-acétique
- Acide (3RS,4RS) ou (3SR,4RS)-4-[3-(R,S)-amino-3-(3-fluoro-6-méthoxyquinolin-4-yl)propyl]-1-[2-(cyclopentylthio)éthyl]pipéridine-3-acétique
- Acide (3RS,4RS) ou (3SR,4RS)-4-[3-(R,S)-amino-3-(3-fluoro-6-méthoxyquinolin-4-yl)propyl]-1-[2-(cyclohexylthio)éthyl]pipéridine-3-acétique
- Acide (3RS,4RS) ou (3SR,4RS)-4-[3-(R,S)-amino-3-(3-fluoro-6-méthoxyquinolin-4-yl)propyl]-1-[2-(5-fluoro-thien-2-yl)thioéthyl]pipéridine-3-acétique
- Acide (3RS,4RS) ou (3SR,4RS)-4-[3-(R,S)-amino-3-(3-fluoro-6-méthoxyquinolin-4-yl)propyl]-1-[2-(thien-3-yl)thioéthyl]pipéridine-3-acétique
- Acide (3RS,4RS) ou (3SR,4RS)-4-[3-(R,S)-amino-3-(3-fluoro-6-méthoxyquinolin-4-yl)propyl]-1-[2-(1,3-thiazol-2-yl)thioéthyl]pipéridine-3-acétique
- Acide (3RS,4RS) ou (3SR,4RS)-4-[3-(R,S)-amino-3-(3-fluoro-6-méthoxyquinolin-4-yl)propyl]-1-[2-(pyridin-2-yl)thioéthyl]pipéridine-3-acétique
- Acide (3RS,4RS) ou (3SR,4RS)-4-[3-(R,S)-amino-3-(3-fluoro-6-méthoxyquinolin-4-yl)propyl]-1-[2-(3-fluoro-pyridin-2-yl)thioéthyl]pipéridine-3-acétique
ainsi que leurs sels.

Les exemples suivants illustrent la présente invention.

### Exemple 1

### Dichlorhydrate de l'acide (3RS, 4RS)-4-[3-(3-fluoro-6-méthoxyquinolin-4-yl)propyl]-1-[2-(thién-2-yl)thioéthyl]-pipéridine-3-acétique.

Une solution de 480 mg de (3RS,4RS)-4-[3-(3-fluoro-6-méthoxyquinolin-4-yl) propyl]-1-[2-(thién-2-yl)thioéthyl]-pipéridine-3-acétate de méthyle, 5 cm³ de dioxane et 2,25 cm³ d'une solution aqueuse d'hydroxyde de sodium 1N, est chauffée sous agitation à une température voisine de 60°C pendant 1 heure 30 minutes. Après concentration du mélange réactionnel sous pression réduite (5 kPa) à une température voisine de 40°C, le résidu obtenu est repris par 50 cm³ d'eau et 20 cm³ d'éther. La phase aqueuse est décantée puis lavée par 2 fois 10 cm³ d'éther. Elle est ensuite acidifiée par une coulée de 2,25 cm³ d'acide chlorhydrique 1N. Le précipité formé est dissout par addition de 75 cm³ de dichlorométhane. La phase organique est décantée, séchée sur sulfate de magnésium, filtrée, puis concentrée sous pression réduite (5 kPa) à une température voisine de 40°C. Le produit obtenu est alors agité dans 30 cm³ d'acétone. On coule sur cette solution 4 cm³ d'acide chlorhydrique 4N dans le dioxane. On concentre le mélange réactionnel sous pression réduite (5 kPa) à une température voisine de 40°C puis on ajoute 15 cm³ d'acétone. On renouvelle cette opération 5 fois, jusqu'à obtention d'un solide jaune que l'on essore, puis que l'on sèche au dessicateur sous pression réduite (10 Pa) à une température d'environ 45°C. On obtient 395 mg de dichlorhydrate de l'acide (3RS,4RS)-4-[3-(3-fluoro-6-méthoxyquinolin-4-yl)propyl]-1-[2-(thién-2-yl)thioéthyl]-pipéridine-3-acétique, sous forme d'un solide de couleur blanc cassé.

Spectre de R.M.N. ¹H (400 MHz, (CD₃)₂SO d6 à une température de 393K, δ en ppm) :
de 1,40 à 1,90 (mt : 7H) ; 2,29 (dd, J = 16 et 5,5 Hz : 1H) ; 2,46 (mf : 1H) ; de 2,65 à 3,45 (mt : 5H) ; 3,09 (t large, J = 7,5 Hz : 2H) ; 3,23 (s large : 4H) ; 3,99 (s : 3H) ; 7,09 (dd, J = 5,5 et 3,5 Hz : 1H) ; 7,27 (dd, J = 3,5 et 1,5 Hz : 1H) ; 7,38 (d, J = 3 Hz : 1H) ; 7,40 (dd, J = 9 et 3 Hz : 1H) ; 7,62 (dd, J = 5,5 et 1,5 Hz : 1H) ; 7,98 (d, J = 9 Hz : 1H) ; 8,64 (s large : 1H).

### (3RS,4RS)-4-[3-(3-fluoro-6-méthoxyquinolin-4-yl) propyl]-1-[2-(2-thiénylthio) éthyl]-pipéridine-3-acétate de méthyle

Une suspension composée de 0,95 g de (3RS, 4RS) et (3SR, 4RS)-4-[3-(3-fluoro-6-méthoxyquinolin-4-yl)propyl]-pipéridine-3-acétate de méthyle, 0,7 g de carbonate de potassium, 0,68 g de 2-(2-bromoéthylthio)thiophène dans 40 cm³ de diméthylformamide est agitée pendant 16 heures à une température voisine de 60°C sous atmosphère inerte. Après refroidissement à environ 20°C, le mélange réactionnel est jeté sur 200 cm³ d'eau et 200 cm³ d'acétate d'éthyle. La phase organique est décantée puis lavée par 5 fois 100 cm³ d'eau puis par 100 cm³ d'une solution aqueuse saturée de chlorure de sodium, séchée sur sulfate de magnésium, filtrée, puis concentrée sous pression réduite (5 kPa) à une température voisine de 40°C. Le résidu d'évaporation obtenu est purifié par chromatographie, sous une pression d'azote de 50 kPa, sur une colonne de gel de silice (granulométrie 20-45µ; diamètre 2 cm; hauteur 40 cm), en éluant par un mélange de cyclohexane-acétate d'éthyle (68/32 en volumes) et en recueillant des fractions de 15 cm³. On concentre les fractions 15 à 21. On obtient 480 mg de (3RS, 4RS)-4-[3-(3-fluoro-6-méthoxyquinolin-4-yl) propyl]-1-[2-(thien-2-yl)thioéthyl]-pipéridine-3-acétate de méthyle, sous forme d'une huile de couleur orange.

Spectre de R.M.N. ¹H (300 MHz, (CD₃)₂SO d6, δ en ppm :
de 1,15 à 1,70 (mt : 7H) ; de 1,90 à 2,05 (mt : 2H) ; 2,08 (mf : 1H); 2,17 (dd large, J = 16 et 4 Hz : 1H) ; de 2,35 à 2,80 (mt : 5H) ; 2,90 (mt : 2H) ; 3,07 (t large, J = 7,5 Hz : 2H) ; 3,57 (s : 3H) ; 3,97 (s : 3H) ; 7,06 (dd, J = 5,5 et 4 Hz : 1H) ; 7,17 (dd, J = 4 et 1,5 Hz : 1H) ; 7,38 (d, J = 3 Hz : 1H) ; 7,40 (dd, J = 9 et 3 Hz : 1H) ; 7,62 (dd, J = 5,5 et 1,5 Hz : 1H) ; 7,97 (d, J = 9 Hz : 1H); 8,70 (s large : 1H).

### (3RS, 4RS) et (3SR, 4RS)-4-[3-(3-Fluoro-6-méthoxyquinolin-4-yl)propyl]-pipéridine-3-acétate de méthyle.

Dans un autoclave, on introduit 2,65 g de (4RS)-1-benzyloxycarbonyl-4-[3-(3-fluoro-6-méthoxyquinolin-4-yl) propyl]-pipéridine-3-ylidène acétate de méthyle, isomère Z, 45 cm³ d'éthanol absolu et 265 mg de palladium sur charbon à 10 %. Le mélange réactionnel est agité sous 5 bars d'hydrogène à 22°C pendant 24 heures, puis, filtré sur supercel, rincé avec 5 fois 20 cm³ d'éthanol absolu. Les filtrats réunis sont concentrés sous pression réduite (5 kPa) à une température voisine de 40°C. On obtient 1,85 g de (3RS, 4RS) et (3SR, 4RS)-4-[3-(3-fluoro-6-méthoxyquinolin-4-yl)propyl]-pipéridine-3-acétate de méthyle, sous forme d'une huile incolore.

Spectre de R.M.N.¹H (300 MHz, (CD₃)₂SO d6, δ en ppm).
de 1,10 à 1,80 (mt : 7H) ; de 1,90 à 2,30 (mt : 2H) ; de 2,35 à 2,60 (mt : 3H) ; de 2,65 à 2,95 (mt : 2H) ; 3,06 (mt : 2H) ; 3,55 et 3,56 (2s : 3H en totalité) ; 3,95 et 3,96 (2s : 3H en totalité) ; de 7,30 à 7,45 (mt : 2H) ; 7,96 (d, J = 9 Hz : 1H); 8,70 (s large : 1H).

### (4RS)-1-benzyloxycarbonyl-4-[3-(3-Fluoro-6-méthoxyquinolin-4-yl) propyl]-pipéridine-3-ylidène acétate de méthyle, isomère Z.

Une solution de 5,8 g de (4RS)-4-allyl-1-benzyloxycarbonyl pipéridin-3-ylidène acétate de méthyle (isomère Z) dans 15 cm³ de tétrahydrofurane est ajoutée lentement, à une température voisine de 0°C, sous agitation et sous atmosphère inerte, à 45 cm³ d'une solution 0,5 M de 9-borabicyclo[3,3,1]nonane dans le tétrahydrofurane. Le mélange est ensuite ramené à une température voisine de 20°C, tandis que l'agitation est poursuivie pendant encore 4 heures. 5,5 g de 4-iodo-3-fluoro-6-méthoxy quinoléine en solution dans 100cm³ de tétrahydrofurane sont ajoutés, puis 11,2 g de phosphate de potassium tribasique, et enfin 386 mg de chlorure de palladium diphénylphosphinoferrocène. Le mélange réactionnel est chauffé pendant 2 heures au reflux puis agité 48 heures à température ambiante. La suspension obtenue est filtrée. Le filtrat est concentré puis repris dans 200 cm³ d'acétate d'éthyle. La solution obtenue est lavée par 2 fois 200 cm³ d'eau puis par 2 fois 200 cm³ d'une solution aqueuse saturée de chlorure de sodium, séchée sur sulfate de magnésium, filtrée, puis concentrée sous pression réduite (5 kPa) à une température voisine de 40°C. On obtient 15 g d'une huile que l'on purifie par chromatographie, sous une pression d'azote de 50 kPa, sur une colonne de gel de silice (granulométrie 20-45µ ; diamètre 6 cm ; hauteur 38 cm), en éluant par un mélange de cyclohexane-acétate d'éthyle (85/15 en volumes en faisant un gradient jusqu'à 70/30 en volumes) et en recueillant des fractions de 200 cm³. Les fractions 31 à 34 sont réunies, puis concentrées. On obtient 4,7 g de (4RS)-1-benzyloxycarbonyl-4-[3-(3-fluoro-6-méthoxyquinolin-4-yl) propyl]-pipéridine-3-ylidène acétate de méthyle (isomère Z), sous forme d'une huile incolore.

Spectre infra rouge (CCl₄): 3091; 3068; 3034; 1705; 1655; 1622; 1507; 1468; 1434; 1361; 1263; 1231; 1207; 1173; 1141; 1034; 909; 832 et 696 cm⁻¹

### (4RS)-4-allyl-1-benzyloxycarbonyl pipéridin-3-ylidène acétate de méthyle, isomère Z.

Une solution contenant 16,3 g de (4RS)-4-allyl-1-benzyloxycarbonyl pipéridin-3-one dans 200 cm³ de toluène est agitée au reflux avec du (triphénylphosphoranylidène) acétate de méthyle, sous atmosphère inerte, pendant 16 heures. Après refroidissement à environ 20°C, le mélange réactionnel est concentré sous pression réduite (5 kPa) à une température voisine de 40°C, le résidu obtenu, solubilisé dans 50 cm³ de dichlorométhane à chaud, est purifié par chromatographie, sous une pression d'azote de 50 kPa, sur une colonne de gel de silice (granulométrie 20-45µ ; diamètre 10 cm; hauteur 45 cm), en éluant par un mélange de cyclohexane-acétate d'éthyle (80/20 en volumes) et en recueillant des fractions de 250 cm³. Les fractions 13 à 15 sont réunies, puis concentrées comme ci-dessus. On obtient 5,8 g de (4RS)-4-allyl-1-benzyloxycarbonyl pipéridin-3-ylidène acétate de méthyle (isomère Z), sous forme d'une huile incolore.

Spectre infra rouge (CCl₄) : 3068; 3034; 2949; 2853; 1722; 1705; 1655; 1643; 1434; 1260; 1200; 1174; 1144; 993; 918 et 696 cm⁻¹

La (4RS)-4-allyl-1-benzyloxycarbonyl-piperidin-3-one peut être préparée selon Takeuchi Y et coll. décrit dans Synthesis 1999, 10, 1814.

### Exemple 2

### Dichlorhydrate de l'acide (3RS,4RS)-4-[3-(R,S)-hydroxy-3-(3-fluoro-6-méthoxyquinolin-4-yl)propyl]-1-[2-(thién-2-ylthio)éthyl]-pipéridine-3-acétique.

Une solution de 70 mg de (3RS,4RS)-4-[3-(R,S)-hydroxy-3-(3-fluoro-6-méthoxyquinolin-4-yl)propyl]-1-[2-(thién-2-ylthio)éthyl]-pipéridine-3-acétate d'éthyle, 1 cm³ de dioxane et 0,3 cm³ d'une solution aqueuse d'hydroxyde de sodium 1N, est chauffée sous agitation à une température voisine de 60°C pendant 1 heure.

Après concentration du mélange réactionnel sous pression réduite (5 kPa) à une température voisine de 40°C, le résidu obtenu est repris par 25 cm³ d'eau et 10 cm³ de dichlorométhane. La phase aqueuse est décantée puis acidifiée par une coulée de 0,3 cm³ d'acide chlorhydrique 1N. Le précipité formé est dissout par addition de 25 cm³ de dichlorométhane. La phase organique est lavée par 10 cm³ d'une solution aqueuse saturée de chlorure de sodium et séchée sur sulfate de magnésium, filtrée, puis concentrée sous pression réduite (5 kPa) à une température voisine de 40°C. Le résidu obtenu est alors dissous à chaud dans 2 cm³ d'acétone. Sur cette solution obtenue, on coule 0,07 cm³ d'acide chlorhydrique 4N dans le dioxane. On concentre le mélange résultant sous pression réduite (5 kPa) à une température voisine de 40°C. On obtient 72 mg du dichlorhydrate de l'acide (3RS,4RS)-4-[3-(R,S)-hydroxy-3-(3-fluoro-6-méthoxyquinolin-4-yl)propyl]-1-[2-(2-thiénylthio)éthyl]-pipéridine-3-acétique, sous forme d'une poudre de couleur blanche.

Spectre de R.M.N.¹H (400 MHz, (CD₃)₂SO d6 avec quelques gouttes de CD3COOD d4 à une température de 373K, δ en ppm) : de 1,20 à 2,00 (mt : 7H) ; de 2,00 à 2,60 (mt : 5H) ; de 2,75 à 3,20 (mt : 6H) ; 3,94 (s : 3H) ; 4,89 (t large, J = 7 Hz : 1H) ; 7,07 (mt : 1H) ; 7,24 (mt : 1H); 7,37 (dd, J = 9 et 2,5 Hz : 1H) ; 7,60 (d large, J = 5 Hz : 1H) ; de 7,90 à 8,00 (mt : 2H) ; 8,62 (s large : 1H).

### (3RS,4RS)-4-[3-(R,S)-hydroxy-3-(3-fluoro-6-méthoxyquinolin-4-yl)propyl]-1-[2-(2-thiénylthio)éthyl]-pipéridine-3-acétate de méthyle.

Une solution composée de 0,92 g de dichlorhydrate de (3RS, 4RS) et (3SR, 4RS)-4-[3-(R,S)-hydroxy-3-(3-fluoro-6-méthoxyquinolin-4-yl)propyl]-pipéridine-3-acétate de méthyle, 0,85 cm³ de triéthylamine, 535 mg de 2-(2-bromoéthylthio)thiophène dans 30 cm³ de diméthylformamide anhydre est agitée pendant 4 heures 30 minutes à une température voisine de 60°C sous atmosphère inerte. On ajoute alors 0,3 cm³ de triéthylamine et on chauffe encore à 60°C sous atmosphère inerte pendant 15 heures. Après refroidissement à environ 20°C, le mélange réactionnel est jeté sur 100 cm³ d'eau et 100 cm³ d'acétate d'éthyle. La phase organique est décantée, lavée par 4 fois 15 cm³ d'eau puis par 2 fois 15 cm³ d'une solution aqueuse saturée de chlorure de sodium, séchée sur sulfate de magnésium, filtrée, concentrée sous pression réduite (5 kPa) à une température voisine de 40°C. Le résidu obtenu est purifié par chromatographie, sous une pression d'azote de 50 kPa, sur une colonne de gel de silice (granulométrie 20-45µ; diamètre 1 cm; hauteur 40 cm), en éluant par un mélange de cyclohexane-acétate d'éthyle (50/50 en volumes) et en recueillant des fractions de 20 cm³. On concentre les fractions 13 à 15. On obtient 70 mg de (3RS, 4RS)-4-[3-(R,S)-hydroxy-3-(3-fluoro-6-méthoxyquinolin-4-yl)-3-(R,S)-hydroxypropyl]-1-[2-(2-thiénylthio)éthyl]-pipéridine-3-acétate de méthyle, sous forme d'une huile de couleur jaune.

Spectre infra rouge (CCl₄) : 3617; 2934; 2799; 2764; 1737; 1623; 1508; 1467; 1231; 1033; 1011; 834 et 698 cm⁻¹

### Dichlorhydrate de (3RS, 4RS) et (3SR, 4RS)-4-[3-(R,S)-hydroxy-3-(3-fluoro-6-méthoxyquinolin-4-yl)propyl]-pipéridine-3-acétate de méthyle.

Une solution de 940 mg d'acide (3RS, 4RS) et (3SR, 4RS)-4-[3-(R,S)-hydroxy-3-(3-fluoro-6-méthoxyquinolin-4-yl)propyl]-1-(tert-butyloxycarbonyl)-pipéridine-3-acétique dans 20 cm³ de méthanol est refroidie à une température voisine de -25°C, sous agitation et sous atmosphère inerte. A cette solution est ajoutée en 5 minutes 0,43 cm³ de chlorure de thionyle. Le mélange est ramené à une température voisine de 20°C, tandis que l'agitation est poursuivie pendant encore 1 heure 30 minutes. On concentre le mélange réactionnel sous pression réduite (5 kPa) à une température voisine de 40°C puis on ajoute 30 cm³ de méthanol. On renouvelle cette série d'opérations 3 fois. On obtient 920 mg de dichlorhydrate de (3RS, 4RS) et (3SR, 4RS)-4-[3-(R,S)-hydroxy-3-(3-fluoro-6-méthoxyquinolin-4-yl)propyl]-pipéridine-3-acétate de méthyle, sous forme d'une meringue jaune.

Spectre infra rouge (KBr) : 3249; 1949; 2503; 2020; 1731; 1622; 1604; 1555; 1497; 1457; 1420; 1308; 1242; 1200; 1175; 1080; 1014; 872; 832 et 795 cm⁻¹

### Acide (3RS, 4RS) et (3SR, 4RS)-4-[3-(R,S)-hydroxy-3-(3-fluoro-6-méthoxyquinolin-4-yl)propyl]-1-(tert-butyloxycarbonyl)-pipéridine-3-acétique.

Une solution de 1,16g de (3RS,4RS) et (3SR,4RS)-4-[3-(3-fluoro-6-méthoxyquinolin-4-yl)-3-propyl]-1-(tert-butyloxycarbonyl)-pipéridine-3-acétate de méthyle, 100 cm³ de diméthylsulfoxyde anhydre et 25 cm³ de tert-butanol anhydre est agitée sous atmosphère inerte exempte d'eau à 20°C. On purge cette solution incolore avec de l'oxygène pur jusqu'à saturation du mélange réactionnel. On ajoute alors une solution contenant 685 mg de tert-butoxyde de potassium dans 8 cm³ de tert-butanol anhydre. L'oxygène est de nouveau introduit par barbotage pendant encore 3 heures et 30 minutes sous forte agitation. La solution jaune obtenue est purgée à l'azote puis refroidie à 0°C. On ajoute ensuite 0,5 cm³ d'acide acétique pur dans 20 cm³ d'eau puis 200 cm³ d'éther. La phase organique est décantée, lavée par 7 fois 20 cm³ d'eau et par 3 fois 20 cm³ d'une solution aqueuse saturée de chlorure de sodium, séchée sur sulfate de magnésium, filtrée et concentrée sous pression réduite (5 kPa) à une température voisine de 40°C. On obtient une gomme que l'on reprend dans 20 cm³ d'éther. On concentre de nouveau dans les mêmes conditions que précédemment. On obtient 945 mg d'acide (3RS,4RS) et (3SR,4RS)-4-[3-(R,S)-hydroxy-3-(3-fluoro-6-méthoxyquinolin-4-yl)propyl]-1-(tert-butyloxycarbonyl)-pipéridine-3-acétique, sous forme d'une meringue blanche.

Spectre infra rouge (KBr) : 2973; 2932; 2864; 1693; 1668; 1623; 1510; 1468; 1429; 1366; 1232; 1166; 1030 et 831 cm⁻¹

Spectre infra rouge (CH₂Cl₂): 3600; 2982; 2939; 2867; 1710; 1682; 1623; 1509; 1468; 1429; 1367; 1231; 1162; 1030; 909; 896 et 834 cm⁻¹

### (3RS, 4RS) et (3SR, 4RS)-4-[3-(3-fluoro-6-méthoxyquinolin-4-yl)-3-propyl]-1-(tert-butyloxycarbonyl )-pipéridine-3-acétate de méthyle.

Une solution de 1,85 g de (3RS,4RS) et (3SR,4RS)-4-[3-(3-fluoro-6-méthoxyquinolin-4-yl)propyl]-pipéridine-3-acétate de méthyle, 0,7 cm³ de triéthylamine et 40 cm³ de dichlorométhane est refroidie, à une température voisine de 0°C, sous agitation et sous atmosphère d'argon. A cette solution incolore est ajoutée en 20 minutes une solution de 1,16 g de di-tert-butyldicarbonate dissous dans 40 cm³ de dichlorométhane. Le mélange est ramené à une température voisine de 20°C, tandis que l'agitation est poursuivie pendant encore 10 heures. On ajoute alors au mélange réactionnel 200 cm³ d'eau. La phase organique est décantée, lavée par 100 cm³ d'une solution aqueuse saturée de chlorure de sodium, séchée sur sulfate de magnésium, filtrée, puis concentrée sous pression réduite (5 kPa) à une température voisine de 40°C. On obtient une huile que l'on purifie par chromatographie, sous une pression d'azote de 50 kPa, sur une colonne de gel de silice (granulométrie 20-45µ ; diamètre 2 cm; hauteur 20 cm), en éluant par un mélange de cyclohexane-acétate d'éthyle (70/30 en volumes) et en recueillant des fractions de 40 cm³. Les fractions 8 à 12 sont réunies, puis concentrées comme précédemment. On obtient 2,16 g de (3RS,4RS) et (3SR,4RS)-4-[3-(3-fluoro-6-méthoxyquinolin-4-yl)-3-propyl]-1-(tert-butyloxycarbonyl)-pipéridine-3-acétate de méthyle, sous forme d'une huile incolore.

Spectre infra rouge (CCl₄) 3006; 1740; 1695; 1622; 1507; 1468; 1428; 1366; 1231; 1166; 1034; 909 et 832 cm⁻¹

### Exemple 3

### Chlorhydrate de l'acide (3SR, 4RS)-4-[3-(3-fluoro-6-méthoxyquinolin-4-yl)propyl]-1-[2-(2-thiénylthio)éthyl]-pipéridine-3-acétique.

Une solution de 195 mg de (3SR,4RS)-4-[3-(3-fluoro-6-méthoxyquinolin-4-yl) propyl]-1-[2-(2-thiénylthio) éthyl]-pipéridine-3-acétate de méthyle, 2 cm³ de dioxane et 0,9 cm³ d'une solution aqueuse d'hydroxyde de sodium 1N, est chauffée sous agitation à une température voisine de 60°C pendant 1 heure. Après concentration du mélange réactionnel sous pression réduite (5 kPa) à une température voisine de 40°C, le résidu obtenu est repris par 20 cm³ d'eau et 10 cm³ d'éther. La phase aqueuse est décantée puis acidifiée par une addition de 0,9 cm³ d'acide chlorhydrique 1N. Le précipité formé est dissout par 20 cm³ de dichlorométhane. La phase organique est lavée par 2 fois 10 cm³ d'une solution aqueuse saturée de chlorure de sodium, séchée sur sulfate de magnésium, filtrée, concentrée sous pression réduite (5 kPa) à une température voisine de 40°C. Le produit obtenu est alors agité dans 20 cm³ d'acétone. On coule sur cette solution une solution de 2 cm³ d'acide chlorhydrique 4N dans le dioxane. On concentre le mélange réactionnel sous pression réduite (5 kPa) à une température voisine de 40°C puis on ajoute 5 cm³ d'acétone. On renouvelle cette opération 4 fois. On obtient 155 mg de chlorhydrate de l'acide (3SR,4RS)-4-[3-(3-fluoro-6-méthoxyquinolin-4-yl)propyl]-1-[2-(2-thiénylthio)éthyl]-pipéridine-3-acétique, sous forme d'un solide de couleur blanc cassé.

Spectre de R.M.N. ¹H (300 MHz, (CD₃)₂SO d6, δ en ppm) : de 1,15 à 2,10 (mt : 8H) ; 2,10 (dd, J = 15 et 7,5 Hz : 1H) ; 2,47 (dd, J = 15 et 4 Hz : 1H) ; de 2,70 à 2,95 (mt : 2H) ; 3,07 (t large, J = 7 Hz : 2H) ; 3,20 (mt : 4H) ; 3,44 (mt : 2H) ; 3,96 (s : 3H) ; 7,11 (dd, J = 5,5 et 4 Hz : 1H) ; 7,32 (dd, J = 4 et 1,5 Hz : 1H) ; 7,40 (mt : 1H) ; 7,41 (dd, J = 9 et 2,5 Hz : 1H) ; 7,72 (dd, J = 5,5 et 1,5 Hz : 1H) ; 7,97 (d, J = 9 Hz : 1H) ; 8,71 (s large : 1H) ; de 9,85 à 10,05 (mf: 1H).

### (3SR,4RS)-4-[3-(3-Fluoro-6-méthoxyquinolin-4-yl) propyl]-1-[2-(2-thiénylthio) éthyl]-pipéridine-3-acétate de méthyle.

Une suspension composée de 0,95 g de (3RS, 4RS) et (3SR, 4RS)-4-[3-(3-fluoro-6-méthoxyquinolin-4-yl)propyl]-pipéridine-3-acétate de méthyle, 0,7 g de carbonate de potassium, 0,68 g de 2-(2-bromoéthylthio)thiophène dans 40 cm³ de diméthylformamide est agitée pendant 16 heures à une température voisine de 60°C sous atmosphère inerte. Après refroidissement à environ 20°C, le mélange réactionnel est additionné sur 200 cm³ d'eau et 200 cm³ d'acétate d'éthyle. La phase organique est décantée puis lavée par 5 fois 100 cm³ d'eau puis par 100 cm³ d'une solution aqueuse saturée de chlorure de sodium, séchée sur sulfate de magnésium, filtrée, concentrée sous pression réduite (5 kPa) à une température voisine de 40°C. Le résidu d'évaporation obtenu est purifié par chromatographie, sous une pression d'azote de 50 kPa, sur une colonne de gel de silice (granulométrie 20-45µ ; diamètre 2 cm; hauteur 40 cm), en éluant par un mélange de cyclohexane-acétate d'éthyle (68/32 en volumes) et en recueillant des fractions de 15 cm³. On concentre les fractions 33 à 36. On obtient 195 mg de (3SR, 4RS)-4-[3-(3-fluoro-6-méthoxyquinolin-4-yl) propyl]-1-[2-(2-thiénylthio) éthyl]-pipéridine-3-acétate de méthyle, sous forme d'une huile de couleur orange.

Spectre de R.M.N. ¹H (300 MHz, (CD₃)₂SO d6, δ en ppm) : de 1,00 à 1,80 (mt : 9H); 1,89 (t trés large, J = 10,5 Hz : 1H) ; 2,07 (dd, J = 15 et 7,5 Hz : 1H) ; de 2,35 à 2,55 (mt : 3H) ; de 2,65 à 2,80 (mt : 2H) ; 2,90 (t, J = 7 Hz : 2H) ; 3,05 (t large, J = 6,5 Hz : 2H) ; 3,56 (s : 3H) ; 3,95 (s : 3H) ; 7,04 (dd, J = 5 et 3,5 Hz : 1H) ; 7,17 (dd, J = 3,5 et 1,5 Hz : 1H) ; 7,37 (mt : 1H) ; 7,40 (dd, J = 9 et 2,5 Hz : 1H) ; 7,60 (dd, J = 5 et 1,5 Hz : 1H) ; 7,96 (d, J = 9 Hz : 1H) ; 8,69 (s large : 1H).

### Exemple 4

### Acide (3RS,4RS)-4-[3-(R,S)-amino-3-(3-fluoro-6-méthoxyquinolin-4-yl)-propyl]-1-[2-(2-thiénylthio)éthyl]-pipéridine 3-acétique

Un mélange de 0,355 g de (3RS,4RS)-4-[3-(R,S)-amino-3-(3-fluoro-6-méthoxyquinolin-4-yl)-propyl]-1-[2-(2-thiénylthio)éthyl]-pipéridine 3-acétate de méthyle dans 1,66 cm³ d'une solution aqueuse de soude 1N et 5 cm³ de dioxane, est porté à une température voisine de 60°C sous agitation pendant 2 heures. Après refroidissement aux environs de 20°C, le mélange réactionnel est concentré à sec à sec sous pression réduite (2 kPa) à une température voisine de 50°C. Le résidu d'évaporation obtenu est repris par 30 cm³ d'eau et 30 cm³ d'éther diéthylique, la phase aqueuse est décantée et neutralisée par 1,66 cm³ d'une solution aqueuse d'acide chlorhydrique 1N et est ensuite extraite par 2 fois 100 cm³ d'acétate d'éthyle. La phase organique est séchée sur du sulfate de magnésium, filtrée puis concentré à sec selon les mêmes conditions précédentes. On obtient 0,238 g d'acide (3RS,4RS)-4-[3-(R,S)-amino-3-(3-fluoro-6-méthoxyquinolin-4-yl)-propyl]-1-[2-(2-thiénylthio)éthyl]-pipéridine 3-acétique, sous forme d'un solide blanc

Spectre de R.M.N. : ¹H (300 MHz, (CD₃)₂SO d6, δ en ppm) : de 0,95 à 2,80 (mts : 16H) ; 2,87 (mt : 2H) ; 3,93 (s : 3H) ; 4,65 (t large, J = 7 Hz : 1H) ; 7,04 (dd large, J = 5,5 et 3,5 Hz : 1H) ; 7,16 (d large, J = 3,5 Hz : 1H) ; 7,37 (d très large, J = 9,5 Hz : 1H); 7,60 (d large, J = 5,5 Hz : 1H); 7,85 (mt : 1 H) ; 7,94 (d, J = 9,5 Hz : 1H); 8,66 (mt : 1H).

### (3RS,4RS)-4-[3-(R,S)-amino-3-(3-fluoro-6-méthoxyquinolin-4-yl)-propyl]-1-[2-(2-thiénylthio)éthyl]-pipéridine 3-acétate de méthyle

A un mélange de 0,98 g de (3RS,4RS)-4-[3-hydroxyimino-3-(3-fluoro-6-méthoxyquinolin-4-yl)-propyl]-1-[2-(2-thiénylthio)éthyl]-pipéridine 3-acétate de méthyle dans 50 cm³ de méthanol refroidi au voisinage de -5°C, sous agitation et atmosphère inerte, on ajoute en plusieurs fois 0,695g de borohydrure de sodium. La réaction est très exothermique et aux environs de 0°C, on ajoute en une seule fois 0,365 g de trioxyde de molybdène. Le mélange réactionnel est agité au voisinage de 20°C pendant 20 heures puis il est refroidi aux environs de -6°C et l'on ajoute de nouveau 0,695 g de borohydrure de sodium et 0,365 g de trioxyde de molybdène. La masse réactionnelle est agitée pendant 5 heures au voisinage de 20°C puis est filtrée sur de la célite et l'insoluble est lavé par 2 fois 50 cm³ de méthanol. Le filtrat est concentré à sec à sec sous pression réduite (2 kPa) à une température voisine de 40°C. Le résidu d'évaporation est purifié par chromatographie sous pression d'argon de 50 kPa, sur une colonne de gel de silice (granulométrie 40-60 µm; diamètre 3 cm ; hauteur 25 cm), en éluant par un mélange successif de cyclohexane-acétate d'éthyle (50/50 en volumes), acétate d'éthyle puis dichlorométhane-méthanol (90/10 en volumes) et en recueillant des fractions de 50 cm³. Les fractions 17 à 20 sont réunies puis concentré à sec selon les conditions décrites précédemment. On obtient 0,355 g de (3RS,4RS)-4-[3-(R,S)-amino-3-(3-fluoro-6-méthoxyquinolin-4-yl)-propyl]-1-[2-(2-thiénylthio)éthyl]-pipéridine 3-acétate de méthyle, sous forme d'une huile incolore.

Spectre infra-rouge : (CC1₄) 2932; 2765; 1736; 1623; 1508; 1230; 1167; 1033; 833 et 699 cm-¹

### (3RS,4RS)-4-[3-hydroxyimino-3-(3-fluoro-6-méthoxyquinolin-4-yl)-propyl)-1-[2-(2-thiénylthio)éthyl]-pipéridine-3-acétate de méthyle

A un mélange de 0,99 g de (3RS,RS)-4-[3-oxo-3-(3-fluoro-6-méthoxyquinolin-4-yl)-propyl)-1-[2-(2-thiénylthio)éthyl]-pipéridine-3-acétate de méthyle dans 10 cm³ de pyridine, sous agitation et atmosphère inerte, on ajoute 0,363 g de chlorhydrate d'hydroxylamine en plusieurs fois et l'ensemble est chauffé au voisinage de 60°C pendant 16 heures. Après refroidissement au voisinage de 20°C, la masse réactionnelle est concentré à sec à sec sous pression réduite (8 kPa) à une température voisine de 55°C. Le résidu d'évaporation est repris par 75 cm³ d'acétate d'éthyle et 40 cm³ d'eau distillée. La phase organique est lavée par 3 fois 40 cm³ d'eau distillée et 40 cm³ d'une solution saturée en chlorure de sodium, séchée sur du sulfate de magnésium pendant 30 minutes, filtrée et concentré à sec sous pression réduite (2 kPa) à une température voisine de 40°C. On obtient 0,98 g de (3RS,4RS)-4-[3-hydroxyimino-3-(fluoro-6-méthoxyquinolin-4-yl)-propyl)-1-[2-(2-thiénylthio)éthyl]-pipéridine-3-acétate de méthyle, sous forme d'une gomme jaune.

| | | |
|---|---|---|
| Spectre de masse : EI m/z=528 [M-OH]⁺ | | |
| m/z=514 | [M-OCH_{3]}⁺ | |
| m/z=416 | [M-C₅H₅S₂]⁺ | pic de base |
| m/z=115 | [C₄H₃S₂]⁺ | |
| DCI m/z=546 MH⁺ | | |

### (3RS,4RS)-4-[3-oxo-3-(3-fluoro-6-méthoxyquinolin-4-yl)-propyl)-1-[2-(2-thiénylthio)éthyl]-pipéridine-3-acétate de méthyle

A une solution de 3,3 cm³ de dichlorure d'oxalyle dans 40 cm³ de dichlorométhane refroidie à -70°C, sous agitation et atmosphère inerte, on verse 4,7 cm³ de diméthylsulfoxyde dans 15cm³ de dichlorométhane en 10 minutes. Au bout de 10 minutes, on verse une solution de 4 g de (3RS,4RS)-4-[3-(R,S)-hydroxy-3-(3-fluoro-6-méthoxyquinolin-4-yl)propyl]-1-[2-(2-thiénylthio)éthyl]-pipéridine 3-acétate de méthyle dans 25 cm³ de dichlorométhane en 10 minutes. Au bout de 20 minutes au voisinage de -70°C, on ajoute goutte à goutte 21 cm³ de triéthylamine dans 20 cm³ de dichlorométhane en 15 minutes et le mélange réactionnel est agité pendant 15 minutes au voisinage de -70°C puis 2 heures au voisinage de 20°C. On verse sur la masse réactionnelle 100 cm³ d'eau distillée, la phase organique est décantée, lavée par 100 cm³ d'une solution aqueuse d'hydrogénocarbonate de sodium saturée, par 2 fois 75 cm³ d'eau et 75 cm³ d'une solution aqueuse de chlorure de sodium saturée. L'extrait organique est séché sur du sulfate de magnésium pendant 30 minutes, filtré et concentré à sec à sec sous pression réduite (2 kPa) à une température voisine de 40°C. Le résidu obtenu est purifié par chromatographie sous pression d'argon de 50 kPa, sur une colonne de gel de silice (granulométrie 40-60 µ; diamètre 3 cm ; hauteur 40 cm), en éluant par un mélange de cyclohexane-acétate d'éthyle (60/40 en volumes) et en recueillant des fractions de 50 cm³. Les fractions 11 à 16 sont réunies puis concentré à sec selon les conditions décrites précédemment. On obtient 3,16 g de (3RS,4RS)-4-[3-oxo-3-(fluoro-6-méthoxyquinolin-4-yl)-propyl)-1-[2-(2-thiénylthio)éthyl]-pipéridine 3-acétate de méthyle, sous forme d'une huile jaune.

Spectre infra-rouge : (CCl₄): 2930 ; 1738; 1699; 1621; 1505; 1231; 1198; 1154; 1028; 834; 699 cm⁻¹.

### Exemple 5

### Chlorhydrate de l'acide (3RS,4RS)-4-[3-(R,S)-hydroxy-3-(3-fluoro-6-méthoxyquinolin-4-yl)-propyl]-1-(2,5-difluoro-phénylthio)-éthyl]-pipéridine-3-acétique

Un mélange de 0,2 g du (3RS,4RS)-4-[3-(R,S)-hydroxy-3-(3-fluoro-6-méthoxyquinolin-4-yl)-propyl]-1-(2,5-difluoro-phénylthio)-éthyl]-pipéridine-3-acétate de méthyle dans 3 cm³ de méthanol, 3 cm³ de dioxane et 1,77 cm³ d'une solution aqueuse de soude 10N est porté à une température voisine de 65°C sous agitation pendant 22 heures. Après refroidissement aux environs de 20°C, le mélange réactionnel est concentré à sec sous pression réduite (2 kPa) à une température voisine de 50°C. Le résidu d'évaporation obtenu est repris par 10 cm³ d'eau distillée et acidifié par un volume suffisant d'une solution d'acide acétique concentrée pour obtenir un pH voisin de 5-6. On extrait par 20 cm³ d'acétate d'éthyle, la phase organique est séchée sur du sulfate de magnésium, filtrée et concentré à sec selon les conditions décrites précédemment. L'huile obtenue est reprise par 10 cm³ d'acétone et 10 cm³ d'une solution de dioxane chlorhydrique puis concentré à sec sous pression réduite (2 kPa) à une température voisine de 40°C. Le résidu est une nouvelle fois repris par 10 cm³ d'acétone puis concentré à sec sous pression réduite (2 kPa) à une température voisine de 40°C puis séché dans un dessiccateur pendant 18 heures. On obtient 0,181 g de chlorhydrate de l'acide (3RS,4RS)-4-[3-(R,S)-hydroxy-3-(3-fluoro-6-méthoxyquinolin-4-yl)-propyl]-1-(2,5-difluoro-phénylthio)-éthyl]-pipéridine-3-acétique, sous forme d'un solide de verseur orange fondant aux environs de 124°C.

Spectre de R.M.N. ¹H (300 MHz, (CD₃)₂SO d6, δ en ppm). On observe un mélange de deux stéréoisomères dans des proportions approximatives 60/40.
* de 1,00 à 3,85 (mts : 18H) ; 3,89 et 4,07 (2s larges : 3H en totalité) ; 5,34 et 5,49 (2 mts : 1H en totalité) ; de 7,05 à 7,55 (mts : 4H) ; de 7,90 à 8,20 (mt : 2H) ; 8,68 et 8,85 (2s larges : 1H en totalité) ; de 9,65 à 10,70 (mfs : 2H en totalité).

### (3RS,4RS)-4-[3-(R,S)-hydroxy-3-(3-fluoro-6-méthoxyquinolin-4-yl)-propyl]-1-[2-(2,5-difluoro-phénylthio)-éthyl]-pipéridine-3-acétate de méthyle

Un mélange de 6,5 g de dichlorhydrate du (3RS,4RS)-4-[3-(R,S)-hydroxy-3-(3-fluoro-6-méthoxyquinolin-4-yl)-propyl]-pipéridine-3-acétate de méthyle, 3,9 g de 2-(2-bromo-éthylthio)-1,4-difluoro-benzène dissout dans 10 cm³ de diméthylformamide, 2,32 g d'iodure de potassium, 5,8 g de carbonate de potassium et 3,93 cm³ de triétylamine dans 200 cm³ d'acétonitrile est chauffé sous agitation et sous atmosphère inerte pendant 22 heures à une température voisine de 70°C. Après refroidissement à une température voisine de 20°C, le mélange réactionnel est filtré et l'insoluble est lavé par 2 fois 30 cm³ d'acétonitrile. Le filtrat est concentré à sec sous pression réduite (2 kPa) à une température voisine de 40°C. Le résidu d'évaporation est repris par 100 cm³ d'eau distillée et 150 cm³ d'acétate d'éthyle. La phase organique est lavée par 3 fois 100 cm³ d'eau distillée et 2 fois 100 cm³ d'une solution aqueuse saturée en chlorure de sodium, séchée sur sulfate de magnésium et concentré à sec selon les conditions décrites précédemment. L'huile obtenue est purifiée par chromatographie sous pression d'argon de 50 kPa, sur une colonne de gel de silice (granulométrie 40-60 µm ; diamètre 4 cm), en éluant par un mélange de cyclohexane-acétate d'éthyle (50/50 en volumes) et en recueillant des fractions de 60 cm³. Les fractions contenant le produit attendu sont réunies puis concentré à sec sous pression réduite (2 kPa) à une température voisine de 40°C. On obtient 1,7 g de (3RS,4RS)-4-[3-(R,S)-hydroxy-3-(3-fluoro-6-méthoxyquinolin-4-yl)-propyl]-1-[2-(2,5-difluorophénylthio)-éthyl]-pipéridine-3-acétate de méthyle, sous forme d'une huile visqueuse de verseur orange.

Spectre de R.M.N. ¹H (300 MHz, (CD₃)₂SO d6, δ en ppm). Nous observons le mélange de deux stéréoisomères dans les proportions 50/50.
* de 0,90 à 2,60 (mt : 14H) ; de 2,60 à 2,80 (mt : 2H) ; 3,08 (t large, J = 7 Hz : 2H) ; 3,47 et 3,55 (2 s : 3H en totalité) ; 3,89 (s : 3H) ; 5,33 (t très large, J = 7 Hz : 1H) ; 5,83 (s large : 1H) ; 7,05 (mt : 1H) ; de 7,15 à 7,35 (mt : 2H) ; 7,38 (d mt, J = 9 Hz : 1H) ; de 7,90 à 8,00 (mt : 2H) ; 8,68 (s large : 1H).

### Exemple 6

### Chlorhydrate de l'acide (3RS,4RS)-4-[3-(R,S)-amino-3-(3-fluoro-6-méthoxyquinolin-4-yl)-propyl]-1-[2-(2,5-difluoro-phénylthio)-éthyl]-pipéridine-3-acétique

Un mélange de 0,09 g de (3RS,4RS)-4-[3-(R,S)-amino-3-(3-fluoro-6-méthoxyquinolin-4-yl)-propyl]-1-[2-(2,5-difluoro-phénylthio)-éthyl]-pipéridine-3-acétate de méthyle dans 0,36 cm³ d'une solution aqueuse de soude 1N et 3 cm³ de dioxane, est porté à une température voisine de 55°C, sous agitation et atmosphère inerte pendant 4 heures. Après refroidissement aux environs de 20°C, le mélange réactionnel est concentré à sec sous pression réduite (2 kPa) à une température voisine de 50°C. Le résidu d'évaporation obtenu est repris par 5 cm³ d'eau distillée et est acidifié par une solution aqueuse d'acide chlorhydrique normal et est concentré N. La phase aqueuse est lavée par 8 cm³ de dichlorométhane puis concentrée selon les mêmes conditions décrites précédemment. On reprend le résidu obtenu par 10 cm³ d'acétone puis on le filtre sur verre fritté. On obtient 0,081 g de chlorhydrate de l'acide (3RS,4RS)-4-[3-(R,S)-amino-3-(3-fluoro-6-méthoxyquinolin-4-yl)-propyl]-1-[2-(2,5-difluoro-phénylthio)-éthyl]-pipéridine-3-acétique, sous forme d'un solide blanc.

Spectre de R.M.N Spectre de R.M.N. ¹H (400 MHz, (CD₃)₂SO d6 avec ajout de quelques gouttes de CD₃COOD d4, à une température de 383K, δ en ppm). Nous observons un mélange de stéréoisomères.
* de 1,20 à 2,55 (mt : 12H) ; de 2,80 à 3,60 (mt : 6H) ; 4,03 (s : 3H) ; 5,16 (mt : 1H) ; 7,12 (mt : 1H) ; 7,25 (mt : 1H) ; 7,42 (mt : 1H) ; 7,49 (d large, J = 9 Hz : 1H) ; 7,54 (mt : 1H) ; 8,06 (d, J = 9 Hz : 1H) ; 8,76 (s large : 1H).

### (3RS,4RS)-4-[3-(RS)-amino-3-(3-fluoro-6-méthoxyquinolin-4-yl)-propyl-1-[2-(2,5-difluoro-phénylthio)-éthyl]-pipéridine-3-acétate de méthyle

A un mélange de 0,4 g de (3RS,4RS)-4-[3-hydroxyimino-3-(3-fluoro-6-méthoxyquinolin-4-yl)-propyl]-1-[2-(2,5-difluoro-phénylthio)-éthyl]-pipéridine-3-acétate de méthyle dans 25 cm³ de méthanol refroidi au voisinage de -2°C, sous agitation et atmosphère inerte, on ajoute en plusieurs fois 0,261 g de borohydrure de sodium. La masse réactionnelle est agitée au voisinage de 0°C pendant 20 minutes puis on ajoute 0,14 g de trioxyde de molybdène. Le mélange réactionnel est agité au voisinage de 20°C pendant 24 heures puis il est refroidi aux environs de -2°C et l'on ajoute de nouveau 0,261 g de borohydrure de sodium et 0,14 g de trioxyde de molybdène. Le mélange réactionnel est agité pendant 18 heures au voisinage de 20°C puis est filtré sur de la célite. Le filtrat est concentré à sec sous pression réduite (2 kPa) à une température voisine de 40°C. Le résidu d'évaporation est purifié par chromatographie sous pression d'argon de 50 kPa, sur une colonne de gel de silice (granulométrie 40-60 µm ; diamètre 3 cm), en éluant par un mélange successif de cyclohexane-acétate d'éthyle (50/50 en volumes) puis dichlorométhane-méthanol (90/10 en volumes) et en recueillant des fractions de 10 cm³. Les fractions 26 à 41 sont réunies puis concentré à sec selon les conditions décrites précédemment. On obtient 0,202 g de (3RS,4RS)-4-[3-(R,S)-amino-3-(3-fluoro-6-méthoxyquinolin-4-yl)-propyl]-1-[2-(2,5-difluoro-phénylthio)-éthyl]-pipéridine-3-acétate de méthyle, sous forme d'une huile incolore.

Spectre infra-rouge : (CCl₄)
2938; 1736; 1623; 1507; 1484; 1230; 1189; 1168; 1033; 909 et 833 cm⁻¹

### (3RS,4RS)-4-[3-hydroxyimino-3-(3-fluoro-6-méthoxyquinolin-4-yl)-propyl]-1-[2-(2,5-difluoro-phénylthio)-éthyl]-pipéridine-3-acétate de méthyle

A un mélange de 1 g de (3RS,4RS)-4-[3-oxo-3-(3-fluoro-6-méthoxyquinolin-4-yl)-propyl)-1-[2-(2,5-difluoro-phénylthio)-éthyl]-pipéridine-3-acétate de méthyle dans 10 cm³ de pyridine, sous agitation et atmosphère inerte, on ajoute 0,347 g de chlorhydrate d'hydroxylamine en plusieurs fois et l'ensemble est agité au voisinage de 20°C pendant 24 heures puis 20 heures au voisinage de 50°C et 1,25 heures au voisinage de 62°C. Après refroidissement au voisinage de 20°C, la masse réactionnelle est concentré à sec à sec sous pression réduite (1,5 kPa) à une température voisine de 50°C. Le résidu d'évaporation est repris par 70 cm³ d'acétate d'éthyle et 40 cm³ d'eau distillée. La phase organique est décantée, lavée par 3 fois 40 cm³ d'eau distillée et 40 cm³ d'une solution saturée en chlorure de sodium, séchée sur du sulfate de magnésium, filtrée et concentré à sec sous pression réduite (2 kPa) à une température voisine de 40°C. Le résidu d'évaporation est purifié par chromatographie sous pression d'argon de 50 kPa, sur une colonne de gel de silice (granulométrie 40-60 µm ; diamètre 3 cm), en éluant par un mélange successif de cyclohexane-acétate d'éthyle (60/40 en volumes) puis dichlorométhane-méthanol (90/10 en volumes) et en recueillant des fractions de 10 cm³. Les fractions 1 à 21 sont réunies puis concentré à sec selon les conditions décrites précédemment. On obtient 0,813 g de (3RS,4RS)-4-[3-hydroxyimino-3-(3-fluoro-6-méthoxyquinolin-4-yl)-propyl]-1-[2-(2,5-difluorophénylthio)-éthyl]-pipéridine-3-acétate de méthyle, sous forme d'une huile visqueuse blanchâtre.

Spectre infra-rouge : (CCl₄)
3585; 3174; 2930; 1738; 1621; 1506; 1484; 1229; 1189; 1167; 1029; 909 et 833 cm⁻¹

### (3RS,4RS)-4-[3-oxo-3-(3-fluoro-6-méthoxyquinolin-4-yl)-propyl)-1-(2-(2,5-difluorophénylthio)-éthyl]-pipéridine-3-acétate de méthyle

A une solution de 1,32 cm³ de dichlorure d'oxalyle dans 30 cm³ de dichlorométhane refroidie à -70°C, sous agitation et atmosphère inerte, on verse 1,88 cm³ de diméthylsulfoxyde dans 6 cm³ de dichlorométhane en 10 minutes. Au bout de 10 minutes, on verse une solution de 1,7 g de (3RS,4RS)-4-[3-(R,S)-hydroxy-3-(3-fluoro-6-méthoxyquinolin-4-yl)-propyl]-1-[2-(2,5-difluoro-phénylthio)-éthyl]-pipéridine-3-acétate de méthyle dans 15 cm³ de dichlorométhane en 10 minutes. Au bout de 15 minutes, on ajoute goutte à goutte 8,4 cm³ de triéthylamine dans 10 cm³ de dichlorométhane en 15 minutes et le mélange réactionnel est agité pendant 45 minutes au voisinage de -70°C puis 20 heures au voisinage de 20°C. On verse sur la masse réactionnelle 50 cm³ d'eau distillée, la phase organique est décantée, lavée par 50 cm³ d'une solution aqueuse d'hydrogénocarbonate de sodium saturée, par 2 fois 30 cm³ d'eau distillée et 30 cm³ d'une solution aqueuse de chlorure de sodium saturée. L'extrait organique est séché sur du sulfate de magnésium, filtré et concentré à sec sous pression réduite (2 kPa) à une température voisine de 40°C. Le résidu obtenu est purifié par chromatographie sous une pression d'argon de 50 kPa, sur une colonne de gel de silice (granulométrie 40-60 µm; diamètre 3 cm), en éluant par un mélange de cyclohexane-acétate d'éthyle (60/40 en volumes) et en recueillant des fractions de 10 cm³. Les fractions contenant le produit attendu sont réunies puis concentré à sec selon les conditions décrites précédemment. On obtient 1,37 g de (3RS,4RS)-4-[3-oxo-3-(3-fluoro-6-méthoxyquinolin-4-yl)-propyl]-1-[2-(2,5-difluoro-phénylthio)-éthyl]-pipéridine-3-acétate de méthyle.

Spectre infra-rouge : (CCl₄) 2930; 1737; 1701; 1621; 1506; 1484; 1468; 1232; 1189; 1166; 1028; 905 et 834 cm⁻¹

### Exemple 7

### Chlorhydrate de l'acide (3RS,4RS)-4-[3-(R,S)-hydroxy-3-(3-fluoro-6-méthoxyquinolin-4-yl)-propyl]-1-(2,5-cyclopentanethio)-éthyl-pipéridine-3-acétique

Un mélange de 0,12 g de (3RS,4RS)-4-[3-(R,S)-hydroxy-3-(3-fluoro-6-méthoxyquinolin-4-yl)-propyl]-1-(2,5-cyclopentanethio)-éthyl-pipéridine-3-acétate de méthyle dans 2 cm³ de dioxane et 0,6 cm³ d'une solution aqueuse de soude 1N est porté à une température voisine de 55°C sous agitation pendant 18 heures. Après refroidissement aux environs de 20°C, le mélange réactionnel est concentré à sec sous pression réduite (2 kPa) à une température voisine de 50°C. Le résidu d'évaporation est repris par 5 cm³ d'eau distillée, acidifié avec un volume suffisant d'une solution aqueuse d'acide chlorhydrique 1N pour obtenir un pH voisin de 6. On extrait par 8 cm³ de dichlorométhane et la phase organique est séchée sur du sulfate de magnésium, filtrée et concentré à sec selon les mêmes conditions précédemment décrites. Le résidu est repris par 5 cm³ d'acétone et 1 cm³ d'une solution d'acide chlorhydrique 4N dans le dioxane puis concentré à sec sous pression réduite (1 kPa) à une température voisine de 50°C. Le résidu est de nouveau repris par 5 cm³ d'acétone et concentré à sec selon les conditions décrites précédemment. On obtient 0,078 g de chlorhydrate de l'acide (3RS,4RS)-4-[3-(R,S)-hydroxy-3-(3-fluoro-6-méthoxyquinolin-4-yl)-propyl]-1-(2,5-cyclopentanethio)-éthyl]-pipéridine-3-acétique, sous forme d'une poudre de verseur orange et fondant aux environs de 125°C.

Spectre de R.M.N. Spectre de R.M.N. ¹H (400 MHz, (CD₃)₂SO d6 avec ajout de quelques gouttes de CD₃COOD d4, à une température de 383K, δ en ppm) : de 1,20 à 2,55 (mt : 18H) ; 2,89 (mt : 2H) ; de 3,00 à 3,35 (mt : 7H) ; 3,94 (s : 3H) ; 5,39 (mt : 1H) ; 7,39 (dd, J = 9 et 2 Hz : 1H) ; 7,97 (d, J = 9 Hz : 1H); 7,99 (mt : 1H) ; 8,63 (s large : 1H).

### (3RS,4RS)-4-[3-(R,S)-hydroxy-3-(3-fluoro-6-méthoxyquinolin-4-yl)-propyl]-1-(2,5-cyclopentanethio)-éthyl-pipéridine-3-acétate de méthyle

Un mélange de 1 g de chlorhydrate de (3RS,4RS)-4-[3-(R,S)-hydroxy-3-(3-fluoro-6-méthoxyquinolin-4-yl)-propyl]-pipéridine-3-acétate de méthyle, 0,424 g de (2-chloroéthylthio)-cyclopentane, 0,388 g d'iodure de potassium, 0,97 g de carbonate de potassium et 0,656 cm³ de triétylamine dans 30 cm³ d'acétonitrile est chauffé sous agitation pendant 18 heures à une température voisine de 65°C. Après refroidissement à une température voisine de 20°C, le mélange réactionnel est filtré et l'insoluble est lavé par 2 fois 20 cm³ d'acétonitrile. Le filtrat est concentré à sec sous pression réduite (1 kPa) à une température voisine de 50°C. Le résidu d'évaporation est repris par 20 cm³ d'eau distillée et 30 cm³ d'acétate d'éthyle. La phase organique est lavée par 2 fois 20 cm³ d'eau distillée et par 2 fois 20 cm³ d'une solution aqueuse saturée en chlorure de sodium, séchée sur sulfate de magnésium, filtrée et concentré à sec selon les conditions décrites précédemment. L'huile obtenue est purifiée par chromatographie sous pression d'argon de 50 kPa, sur une colonne de gel de silice (granulométrie 40-60 µm; diamètre 3 cm), en éluant par un mélange successif de cyclohexane-acétate d'éthyle (60/40 en volumes) puis dichlorométhane-méthanol (90/10 en volumes) et en recueillant des fractions de 6 cm³. Les fractions 6 à 25 sont réunies puis concentré à sec sous pression réduite (2 kPa) à une température voisine de 40°C. On obtient 0,42 g de (3RS,4RS)-4-[3-(R,S)-hydroxy-3-(3-fluoro-6-méthoxyquinolin-4-yl)-propyl]-1-[2-(cyclopentanethio)-éthyl]-pipéridine-3-acétate de méthyle, sous forme d'une huile jaune.

Spectre infra-rouge : (CCl₄)
3616; 2928; 2853; 1737; 1623; 1508; 1231; 1165; 1032; 907 et 834 cm⁻¹

La préparation du chlorhydrate (3RS,4RS)-4-[3-(R,S)-hydroxy-3-(3-fluoro-6-méthoxyquinolin-4-yl)]-propyl-pipéridine-3-acétate de méthyle a été décrite ci-avant.

### Exemple 8

### Synthèse de stéréoisomères de l'acide 4-[3-hydroxy-3-(3-fluoro-6-méthoxyquinolin-4-yl)propyl]-1-[2-(2-thiénylthio)-éthyl]-pipéridine-3-acétique

La stéréochimie absolue de chaque stéréoisomère ci-après appelée I, II, ID, IV, V, VI, VII n'est pas connue.

### Stéréoisomère I

Une solution de 0,625 g du 4-[3-hydroxy-3-(3-fluoro-6-méthoxyquinolin-4-yl)propyl]-1-[2-(2-(thiènylthio)-éthyl)]-pipéridine-3-acétate de méthyle (stéréoisomère I) dans 10 cm³ de dioxanne et 3 cm³ d'une solution aqueuse d'hydroxyde de sodium 1N, est chauffée sous agitation à une température voisine de 60°C pendant 1 heure. Après concentration du mélange réactionnel sous pression réduite (5 kPa) à une température voisine de 40°C, le résidu obtenu est repris par 10 cm³ d'eau, acidifié par une solution aqueuse d'acide chlorhydrique 1N. Le précipité formé est filtré, séché à l'étuve sous une pression réduite (10 Pa) à une température voisine de 20°C pendant 18 heures. Le produit obtenu est ensuite agité dans 30 cm³ d'acétone et l'on verse une solution d'acide chlorhydrique 4N dans le dioxane. Le mélange réactionnel est concentré sous pression réduite (5 kPa) à une température voisine de 40°C puis on reprend le produit par de l'acétone 3 fois en évaporant selon les conditions précédemment décrites, à chaque étape. On obtient 0,42 g de dichlorhydrate de l'acide 4-[3-hydroxy-3-(3-fluoro-6-méthoxyquinolin-4-yl)propyl]-1-[2-(2-thiénylthio)-éthyl]-pipéridine-3-acétique (stéréoisomère I) sous la forme d'un solide blanc. (α_{D}²⁰= +56,9°+/-1,0 dans le méthanol à 0,5 %).

Spectre de R.M.N ¹H (400 MHz, (CD₃)₂SO d6 avec ajout de quelques gouttes de CD₃COOD d4, à une température de 373K, δ en ppm) : de 1,25 à 2,65 (mt : 9H) ; 2,33 (dd, J = 15 et 5 Hz : 1H) ; de 3,05 à 3,40 (mt : 8H) ; 3,94 (s : 3H) ; 5,40 (t large, J = 7 Hz : 1H) ; 7,09 (mt : 1H) ; 7,28 (d large, J = 3 Hz : 1H) ; 7,37 (d très large, J = 9 Hz : 1H) ; 7,62 (d large, J = 5 Hz : 1H) ; de 7,90 à 8,00 (mt : 2H) ; 8,61 (s large : 1H).

### Stéréoisomère II

Une solution de 0,545 g 4-[3-hydroxy-3-(3-fluoro-6-méthoxyquinolin-4-yl)propyl]-1-[2-(2-(thiènylthio)-éthyl)]-pipéridine-3-acétate de méthyle (stéréoisomère II) dans 10 cm³ de dioxanne et 2,6 cm³ d'une solution aqueuse d'hydroxyde de sodium 1N, est chauffée sous agitation à une température voisine de 60°C pendant 1 heure. Après concentration du mélange réactionnel sous pression réduite (5 kPa) à une température voisine de 40°C, le résidu obtenu est repris par 10 cm³ d'eau, acidifié par une solution aqueuse d'acide chlorhydrique 1N afin d'obtenir un pH égal à 6. La suspension est reprise par 20 cm³ de dichlorométhane puis la phase organique est décantée, séchée sur du sulfate de magnésium, filtrée, concentrée sous pression réduite (5 kPa) à une température voisine de 40°C. Le produit obtenu est alors agité dans 20 cm³ d'acétone et l'on verse une solution d'acide chlorhydrique 4N dans le dioxanne. Le précipité formé est filtré, séché à l'étuve sous une pression réduite (10 Pa) à une température voisine de 20°C pendant 18 heures. Le résidu obtenu est ensuite repris par de l'acétone plusieurs fois en évaporant selon les conditions précédemment décrites, à chaque étape. On obtient 0,43 g de dichlorhydrate de l'acide 4-[3-hydroxy-3-(3-fluoro-6-méthoxyquinolin-4-yl)propyl]-1-[2-(2-thiènylthio)-éthyl]-pipéridine-3-acétique (stéréoisomère II) sous la forme d'un solide blanc. (α_{D}²⁰= +55,9°+/-0,9 dans le méthanol à 0,5 %).

Spectre de R.M.N ¹H (400 MHz, (CD₃)₂SO d6 avec ajout de quelques gouttes de CD₃COOD d4, à une température de 373K, δ en ppm) : de 1,25 à 2,60 (mt : 10H) ; de 3,00 à 3,40 (mt : 8H) ; 3,94 (s : 3H) ; 5,39 (dd, J = 7 et 5 Hz : 1H) ; 7,09 (dd, J = 5 et 3 Hz : 1H) ; 7,28 (d large, J = 3 Hz : 1H) ; 7,38 (dd, J = 9 et 2,5 Hz : 1H) ; 7,63 (d large, J = 5 Hz : 1H) ; de 7,90 à 8,05 (mt : 2H) ; 8,63 (s large : 1H).

### Stéréoisomère III

Une solution de 0,458 g de 4-[3-hydroxy-3-(3-fluoro-6-méthoxyquinolin-4-yl)propyl]-1-[2-(2-(thiènylthio)-éthyl)]-pipéridine-3-acétate de méthyle (stéréoisomère III) dans 10 cm³ de dioxanne et 2,2 cm³ d'une solution aqueuse d'hydroxyde de sodium 1N, est chauffée sous agitation à une température voisine de 60°C pendant 1 heure. Après concentration du mélange réactionnel sous pression réduite (5 kPa) à une température voisine de 40°C, le résidu obtenu est repris par 10 cm³ d'eau, acidifié par une solution aqueuse d'acide acétique afin d'obtenir un pH égal à 6. La suspension est reprise par 20 cm³ de dichlorométhane puis la phase organique est décantée, séchée sur du sulfate de magnésium, filtrée, concentrée sous pression réduite (5 kPa) à une température voisine de 40°C. Le produit obtenu est alors agité dans 20 cm³ d'acétone et l'on verse une solution d'acide chlorhydrique 4N dans le dioxane. Le précipité formé est filtré, séché à l'étuve sous une pression réduite (10 Pa) à une température voisine de 20°C pendant 18 heures. Le résidu obtenu est ensuite repris par de l'acétone plusieurs fois en évaporant selon les conditions précédemment décrites, à chaque étape. On obtient 0,42 g de dichlorhydrate de l'acide 4-[3-hydroxy-3-(3-fluoro-6-méthoxyquinolin-4-yl)propyl]-1-[2-(2-thiénylthio)-éthyl]-pipéridine-3-acétique (stéréoisomère III) sous la forme d'un solide blanc. (α_{D}²⁰= -46,9°+/-0,9 dans le méthanol à 0,5 %).

Spectre de R.M.N ¹H (400 MHz, (CD₃)₂SO d6 avec ajout de quelques gouttes de CD₃COOD d4, à une température de 373K, δ en ppm): de 1,25 à 2,60 (mt : 10H); de 3,00 à 3,40 (mt : 8H) ; 3,94 (s : 3H) ; 5,39 (t large, J = 7 Hz : 1H); 7,09 (mt : 1H); 7,28 (d large, J = 3 Hz : 1H); 7,38 (d très large, J = 9 Hz : 1H); 7,63 (d large, J = 5 Hz : 1H); de 7,90 à 8,05 (mt : 2H) ; 8,62 (s large : 1H).

### Stéréoisomère IV

Une solution de 0,454 g du 4-[3-hydroxy-3-(3-fluoro-6-méthoxyquinolin-4-yl)propyl]-1-[2-(2-(thiènylthio)-éthyl)]-pipéridine-3-acétate de méthyle (stéréoisomère IV) dans 10 cm³ de dioxanne et 2,2 cm³ d'une solution aqueuse d'hydroxyde de sodium 1N, est chauffée sous agitation à une température voisine de 60°C pendant 1 heure. Après concentration du mélange réactionnel sous pression réduite (5 kPa) à une température voisine de 40°C, le résidu obtenu est repris par 10 cm³ d'eau, acidifié par une solution aqueuse d'acide acétique afin d'obtenir un pH égal à 6. La suspension est reprise par 20 cm³ de dichlorométhane puis la phase organique est décantée, séchée sur du sulfate de magnésium, filtrée, concentrée sous pression réduite (5 kPa) à une température voisine de 40°C. Le produit obtenu est alors agité dans 20 cm³ d'acétone et l'on verse une solution d'acide chlorhydrique 4N dans le dioxane. Le précipité formé est filtré, séché à l'étuve sous une pression réduite (10 Pa) à une température voisine de 20°C pendant 18 heures. Le résidu obtenu est ensuite repris par de l'acétone plusieurs fois en évaporant selon les conditions précédemment décrites, à chaque étape. On obtient 0,35 g de dichlorhydrate de l'acide 4-[3-hydroxy-3-(3-fluoro-6-méthoxyquinolin-4-yl)propyl]-1-[2-(2-thiénylthio)-éthyl]-pipéridine-3-acétique (stéréoisomère IV) sous la forme d'un solide blanc. (α_{D}²⁰= -54,8°+/-1,1 dans le méthanol à 0,5 %).

Spectre de R.M.N ¹H (400 MHz, (CD₃)₂SO d6 avec ajout de quelques gouttes de CD₃COOD d4, à une température de 373K, δ en ppm) : de 1,25 à 2,60 (mt : 9H) ; 2,22 (dd large, J = 15 et 5 Hz : 1H) ; de 3,00 à 3,40 (mt : 8H) ; 3,94 (s : 3H) ; 5,40 (mt : 1H) ; 7,10 (mt : 1H) ; 7,29 (d large, J = 3 Hz : 1H) ; 7,38 (d très large, J = 9 Hz : 1H) ; 7,64 (d large, J = 5 Hz : 1H) ; de 7,90 à 8,05 (mt : 2H) ; 8,62 (s large : 1H).

### Stéréoisomère V

Une solution de 0,560 g du 4-[3-hydroxy-3-(3-fluoro-6-méthoxyquinolin-4-yl)propyl]-1-[2-(2-(thiènylthio)-éthyl)]-pipéridine-3-acétate de méthyle (stéréoisomère V) dans 10 cm³ de dioxanne et 2,2 cm³ d'une solution aqueuse d'hydroxyde de sodium 1N, est chauffée sous agitation à une température voisine de 60°C pendant 2 heures. Après concentration du mélange réactionnel sous pression réduite (5 kPa) à une température voisine de 40°C, le résidu obtenu est repris par 10 cm³ d'eau, acidifié par une solution aqueuse d'acide acétique afin d'obtenir un pH égal à 6. La suspension est reprise par 20 cm³ de dichlorométhane puis la phase organique est décantée, séchée sur du sulfate de magnésium, filtrée, concentrée sous pression réduite (5 kPa) à une température voisine de 40°C. Le produit obtenu est alors agité dans 20 cm³ d'acétone et l'on verse une solution d'acide chlorhydrique 4N dans le dioxane. Le précipité formé est concentré sous pression réduite (5 kPa) à une température voisine de 40°C. Le résidu obtenu est ensuite repris par de l'acétone plusieurs fois en évaporant selon les conditions précédemment décrites, à chaque étape puis séché à l'étuve sous une pression réduite (10 Pa) à une température voisine de 20°C pendant 120 heures. On obtient 0,4 g de dichlorhydrate de l'acide 4-[3-hydroxy-3-(3-fluoro-6-méthoxyquinolin-4-yl)propyl]-1-[2-(2-thiénylthio)-éthyl]-pipéridine-3-acétique (stéréoisomère V) sous la forme d'un solide blanc. (α_{D}²⁰= +83,4°+/-1,3 dans le méthanol à 0,5 %).

Spectre de R.M.N. ¹H (400 MHz, (CD₃)₂SO d6, δ en ppm) : 0,95 (mt : 1H) ; 1,41 (mt : 2H) ; de 1,65 à 2,15 (mt : 6H) ; 2,36 (dd, J = 16,5 et 3,5 Hz : 1H) ; de 2,70 à 2,90 (mt : 2H) ; 3,13 (mt : 2H) ; 3,20 (mt : 2H) ; de 3,35 à 3,55 (mt : 2H) ; 3,91 (s : 3H) ; 5,32 (t large, J = 7,5 Hz : 1H) ; 7,10 (dd, J = 5,5 et 3,5 Hz : 1H) ; 7,28 (dd, J = 3,5 et 1 Hz : 1H) ; 7,38 (dd, J = 9 et 3 Hz : 1H) ; 7,68 (dd, J = 5,5 et 1 Hz : 1H) ; 7,94 (mt: 1H) ; 7,96 (d, J = 9 Hz : 1H) ; 8,70 (d, J = 1,5 Hz : 1H) ; 10,15 (mf: 1H).

### Stéréoisomère VI

Une solution de 0,36 g du 4-[3-hydroxy-3-(3-fluoro-6-méthoxyquinolin-4-yl)propyl]-1-[2-(2-(thiènylthio)-éthyl)]-pipéridine-3-acétate de méthyle (stéréoisomère VI) dans 10 cm³ de dioxanne et 1,7 cm³ d'une solution aqueuse d'hydroxyde de sodium 1N, est chauffée sous agitation à une température voisine de 60°C pendant 2 heures. Après concentration du mélange réactionnel sous pression réduite (5 kPa) à une température voisine de 40°C, le résidu obtenu est repris par 10 cm³ d'eau, acidifié par une solution aqueuse d'acide acétique afin d'obtenir un pH égal à 6. La suspension est reprise par 2 fois 10 cm³ de dichlorométhane puis la phase organique est décantée, séchée sur du sulfate de magnésium, filtrée, concentrée sous pression réduite (5 kPa) à une température voisine de 40°C. Le produit obtenu est alors agité dans 20 cm³ d'acétone et l'on verse une solution d'acide chlorhydrique 4N dans le dioxane. Le précipité formé est concentré sous pression réduite (5 kPa) à une température voisine de 40°C. Le résidu obtenu est ensuite repris par de l'acétone plusieurs fois en évaporant selon les conditions précédemment décrites, à chaque étape puis séché à l'étuve sous une pression réduite (10 Pa) à une température voisine de 20°C pendant 48 heures. On obtient 0,325 g de dichlorhydrate de l'acide 4-[3-hydroxy-3-(3-fluoro-6-méthoxyquinolin-4-yl)propyl]-1-[2-(2-thiénylthio)-éthyl]-pipéridine-3-acétique (stéréoisomère VI) sous la forme d'un solide blanc. (α_{D}²⁰= +29,8°+/-0,8 dans le méthanol à 0,5 %).

Spectre de R.M.N. ¹H (300 MHz, (CD₃)₂SO d6, δ en ppm) : de 1,30 à 2,20 (mt : 9H) ; 2,46 (mt : 1H) ; de 2,70 à 2,95 (mt : 2H) ; de 3,10 à 3,30 (mt : 4H) ; de 3,35 à 3,65 (mt : 2H); 3,91 (s : 3H) ; 5,32 (dd, J = 8 et 6 Hz : 1H); 7,12 (dd, J = 5,5 et 3,5 Hz : 1H); 7,31 (dd, J = 3,5 et 1Hz : 1H); 7,40 (dd, J = 9 et 3 Hz : 1H); 7,72 (dd, J = 5,5 et 1Hz: 1H); 7,96 (d, J = 9 Hz : 1H); 7,98 (mt : 1H); 8,70 (d, J = 1,5 Hz : 1H); 9,93 (mf: 1H).

### Stéréoisomère VII

Une solution de 0,39 g du 4-[3-hydroxy-3-(3-fluoro-6-méthoxyquinolin-4-yl)propyl]-1-[2-(2-(thiènylthio)-éthyl)]-pipéridine-3-acétate de méthyle (stéréoisomère VII) dans 10 cm³ de dioxanne et 1,8 cm³ d'une solution aqueuse d'hydroxyde de sodium 1N, est chauffée sous agitation à une température voisine de 60°C pendant 2 heures. Après concentration du mélange réactionnel sous pression réduite (5 kPa) à une température voisine de 40°C, le résidu obtenu est repris par 10 cm³ d'eau, acidifié par une solution aqueuse d'acide acétique afin d'obtenir un pH égal à 6. La suspension est reprise par 20 cm³ de dichlorométhane puis la phase organique est décantée, séchée sur du sulfate de magnésium, filtrée, concentrée sous pression réduite (5 kPa) à une température voisine de 40°C. Le produit obtenu est alors agité dans 20 cm³ d'acétone et l'on verse une solution d'acide chlorhydrique 4N dans le dioxane. Le précipité formé est concentré sous pression réduite (5 kPa) à une température voisine de 40°C. Le résidu obtenu est ensuite repris par de l'acétone plusieurs fois en évaporant selon les conditions précédemment décrites, à chaque étape puis séché à l'étuve sous une pression réduite (10 Pa) à une température voisine de 20°C pendant 48 heures. On obtient 0,4 g de dichlorhydrate de l'acide 4-[3-hydroxy-3-(3-fluoro-6-méthoxyquinolin-4-yl)propyl]-1-[2-(2-thiénylthio)-éthyl]-pipéridine-3-acétique (stéréoisomère VII) sous la forme d'un solide blanc. (α_{D}²⁰= -27,1°+/-0,7 dans le méthanol à 0,5 %).

Spectre de R.M.N. ¹H (300 MHz, (CD₃)₂SO d6, δ en ppm) : de 1,30 à 2,20 (mt : 9H) ; 2,45 (mt : 1H) ; de 2,70 à 2,95 (mt : 2H) ; de 3,10 à 3,30 (mt : 4H) ; de 3,35 à 3,70 (mt : 2H) ; 3,91 (s : 3H) ; 5,32 (dd, J = 8 et 6 Hz : 1H) ; 7,10 (dd, J = 5,5 et 3,5 Hz : 1H) ; 7,30 (dd, J = 3,5 et 1Hz : 1H) ; 7,39 (dd, J = 9 et 3 Hz : 1H) ; 7,71 (dd, J = 5,5 et 1Hz : 1H) ; 7,95 (d, J = 9 Hz : 1H) ; 7,97 (mt : 1H) ; 8,68 (d, J = 1,5 Hz : 1H) ; 9,96 (mf: 1H).

### Synthèse de stéréoisomères de 4-[3-hydroxy-3-(3-fluoro-6-méthoxyquinolin-4-yl)propyl]-1-[2-(2-(thiènylthio)-éthyl)]-pipéridine-3-acétate de méthyle

Un mélange de 11,9 g de dichlorhydrate de 4-[3-hydroxy-3-(3-fluoro-6-méthoxyquinolin-4-yl)propyl]-pipéridine-3-acétate de méthyle, 4,5 g de 2-(2-bromoéthylthio)thiophène, 3 g d'iodure de potassium, 7,5 g de carbonate de potassium et 5 cm³ de triétylamine dans 200 cm³ d'acétonitrile et 100 cm3 de diméthylformamide est chauffé sous agitation pendant 16 heures à une température voisine de 65°C. Le mélange réactionnel est filtré. Le filtrat est concentré à sec sous pression réduite (1 kPa) à une température voisine de 50°C. Le résidu d'évaporation est purifié par chromatographie sous pression d'argon de 40 kPa, sur une colonne de gel de silice (granulométrie 40-60 µm ; diamètre 8 cm, hauteur 40 cm), en éluant par un mélange de cyclohexane-acétate d'éthyle (50/50 en volumes) en recueillant des fractions de 200 cm³après le passage de 3 dm³ d'un mélange de cyclohexane-acétate d'éthyle (50/50 en volumes). Les fractions 20 à 40 sont réunies puis concentré à sec sous pression réduite (2 kPa) à une température voisine de 40°C pour obtenir 3,1 g de mélange des stéréoisomères I, II, III, IV, sous forme d'une huile jaune et les fractions 48 à 90 sont réunies puis concentré à sec sous pression réduite (2 kPa) à une température voisine de 40°C pour obtenir 3,8 g de mélange des stéréoisomères V, VI, VII, VIII, sous forme d'une huile jaune

### La stéréochimie absolue de chaque stéréoisomère (esters) ci-après appelé I, II, III, IV V, VI, VII. VIII n'est pas connue.

A partir du mélange de stéréoisomères I,II,III,IV obtenus précédemment, la séparation de chaque stéréoisomère s'effectue par HPLC :

La séparation des 2 couples de stéréoisomères (I+II) et (III+IV), est réalisée sur une phase stationnaire chirale à partir de 2,7 g du mélange I,II,III,IV décrit précédemment dans l'exemple 2, (type de phase : chiracel OD ; granulométrie 20 µmm ; diamètre 80 mm ; masse de la phase stationnaire 1,2 kg), sous une pression de 600 kPa, la phase mobile est composée d'un mélange de heptane-propanol-2 (90/10 en volumes) ayant un débit de 160 cm³ par minute et la longueur d'onde du détecteur UV est fixée à 265 nm. Les fractions contenant la première paire de diastéréoismères notée (I+II) sont réunies et concentré à sec sous pression réduite (2 kPa) à une température voisine de 40°C et l'on en obtient 1,3 g sous forme d'une huile avec un taux de récupération égal à 96%. Les fractions contenant la deuxième paire de diastéréoismères notée (III+IV) sont réunies et concentré à sec sous pression réduite (2 kPa) à une température voisine de 40°C et l'on en obtient 1,03 g sous forme d'une huile avec un taux de récupération égal à 76 %. Ensuite les produits de chaque couple de stéréoisomères (I-II et III-IV) sont séparés sur une colonne chiralpak AD (granulométrie 20 µmm ; diamètre 80 mm ; masse de la phase stationnaire 1,2 kg) sous une pression de 800 kPa, la phase mobile est composée d'un mélange de heptane-éthanol (90/10 en volumes) ayant un débit de 200 cm³ par minute et la longueur d'onde du détecteur UV est fixée à 280 nm. Les fractions contenant chaque produit sont isolées puis concentrées sous une pression réduite (2 kPa) à une température voisine de 40°C ; c'est ainsi que l'on obtient 0,632 g du stéréoisomère 1, 0,553 g du stéréoisomère II, 0,463 g du stéréoisomère III, 0,46 g du stéréoisomère IV.

Spectre de R.M.N du stéréoisomère I : ¹H (300 MHz, (CD₃)₂SO d6, δ en ppm) : de 0,85 à 1,55 (mt : 6H) ; de 1,75 à 2,10 (mt : 6H) ; de 2,35 à 2,55 (mt : 2H) ; de 2,55 à 2,80 (mt : 2H) ; 2,87 (t, J = 7 Hz : 2H) ; 3,50 (s : 3H) ; 3,89 (s : 3H) ; 5,33 (t large, J = 7 Hz : 1H) ; 5,82 (s large : 1H) ; 7,04 (dd, J = 5,5 et 3,5 Hz : 1H) ; 7,15 (dd, J = 3,5 et 1,5 Hz : 1H) ; 7,38 (dd, J = 9 et 3 Hz : 1H) ; 7,60 (dd, J = 5,5 et 1,5 Hz : 1H) ; de 7,90 à 8,00 (mt : 2H) ; 8,68 (d, J = 2 Hz : 1H).
Condition HPLC : colonne Chiralpack^{®},débit 1 cm³/min, condition d'élution
de 0 à 13 mim : éthanol-heptane (7/93 en volumes)
de 13 à 28 min (en gradient) éthanol-heptane (15/93 en volumes)
de 28 à 35 min (en gradient) éthanol-heptane (7/93 en volumes)
Temps de rétention : 24,13 min

Spectre de R.M.N du stéréoisomère II : ¹H (300 MHz, (CD₃)₂SO d6, δ en ppm) : de 1,00 à 1,55 (mt : 6H) ; de 1,70 à 2,15 (mt : 5H); 2,21 (dd, J = 16 et 3 Hz : 1H); de 2,30 à 2,60 (mt : 2H); 2,67 (mt : 2H) ; 2,89 (t, J = 7 Hz : 2H) ; 3,59 (s : 3H) ; 3,90 (s : 3H) ; 5,34 (mt : 1H) ; 5,83 (d très large, J = 3 Hz : 1H) ; 7,05 (dd, J = 5,5 et 3,5 Hz : 1H) ; 7,17 (dd large, J = 3,5 et 1,5 Hz : 1H) ; 7,39 (dd, J = 9 et 3 Hz : 1H) ; 7,61 (dd, J = 5,5 et 1,5 Hz : 1H) ; 7,96 (d, J = 9 Hz : 1H) ; 7,99 (mt : 1H) ; 8,69 (s large : 1H).
Condition HPLC : colonne Chiralpack^{®},débit 1 cm³/min, condition d'élution
de 0 à 13 mim : éthanol-heptane (7/93 en volumes)
de 13 à 28 min ( en gradient) éthanol-heptane (15/93 en volumes)
de 28 à 35 min ( en gradient) éthanol-heptane (7/93 en volumes)
Temps de rétention : 29,04 min

Spectre de R.M.N du stéréoisomère III : ¹H (300 MHz, (CD₃)₂SO d6, δ en ppm) : de 1,00 à 1,55 (mt : 6H) ; de 1,70 à 2,15 (mt : 5H) ; 2,21 (dd, J = 16 et 3,5 Hz : 1H) ; de 2,30 à 2,60 (mt : 2H) ; 2,67 (mt : 2H) ; 2,88 (t, J = 7 Hz : 2H) ; 3,58 (s : 3H) ; 3,90 (s : 3H) ; 5,33 (mt : 1H) ; 5,82 (s large : 1H) ; 7,04 (dd, J = 5,5 et 3,5 Hz : 1H) ; 7,16 (dd, J = 3,5 et 1,5 Hz : 1H) ; 7,38 (dd, J = 9 et 3 Hz : 1H) ; 7,59 (dd, J = 5,5 et 1,5 Hz : 1H) ; 7,96 (d, J = 9 Hz : 1H) ; 7,97 (mt : 1H) ; 8,68 (d large, J = 1,5 Hz : 1H).
Condition HPLC : colonne Chiralpack^{®},débit 1 cm³/min, condition d'élution
de 0 à 13 mim : éthanol-heptane (7/93 en volumes)
de 13 à 28 min (en gradient) éthanol-heptane (15/93 en volumes)
de 28 à 35 min (en gradient) éhanol-heptane (7/93 en volumes)
Temps de rétention : 23 min

Spectre de R.M.N du stéréoisomère IV : ¹H (300 MHz, (CD₃)₂SO d6, δ en ppm) : de 0,90 à 1,55 (mt : 6H) ; de 1,75 à 2,10 (mt : 6H) ; de 2,35 à 2,55 (mt : 2H) ; de 2,55 à 2,80 (mt : 2H) ; 2,87 (t, J = 7 Hz : 2H) ; 3,50 (s : 3H) ; 3,90 (s : 3H) ; 5,35 (mt : 1H) ; 5,83 (d, J = 3 Hz : 1H) ; 7,04 (dd, J = 5,5 et 3,5 Hz : 1H) ; 7,15 (dd large, J = 3,5 et 1,5 Hz : 1H) ; 7,38 (dd, J = 9 et 3 Hz : 1H) ; 7,60 (dd large, J = 5,5 et 1,5 Hz : 1H) ; de 7,90 à 8,00 (mt : 2H) ; 8,69 (s large : 1H).
Condition HPLC : colonne Chiralpack^{®},débit 1 cm³/min, condition d'élution
de 0 à 13 mim : éthanol-heptane (7/93 en volumes)
de 13 à 28 min (en gradient) éthanol-heptane (15/93 en volumes)
de 28 à 35 min (en gradient) éthanol-heptane (7/93 en volumes)
Temps de rétention : 25,38 min

### A partir du mélange de stéréoisomères V,VI,VII,VIII obtenus précédemment, la séparation de chaque stéréoisomère s'effectue par HPLC :

La séparation des 2 couples de stéréoisomères est réalisée sur une phase stationnaire C18 à partir de 3,5 g du mélange V,VI,VII,VIII décrit précédemment, (type de phase : KROMACIL^{®} C 18 ; granulométrie 7 µm; diamètre 4,6 mm ; masse de la phase stationnaire 1 kg), sous une pression de 5000 kPa, la phase mobile est composée d'un mélange de eau-acétonitrile-méthanol-acide trifluoroacétique (60/15/25/0.05 en volumes) ayant un débit de 140 cm³ par minute et la longueur d'onde du détecteur UV est fixée à 254 nm. Les fractions contenant la première paire de stéréoisomères notée (V+VIII) sont réunies et concentrés à sec sous pression réduite (2 kPa) à une température voisine de 40°C et l'on en obtient 1,84 g sous forme d'une huile. Les fractions contenant la deuxième paire de stéréoisomères notée (VI+VII) sont réunies et concentré à sec sous pression réduite (2 kPa) à une température voisine de 40°C et l'on en obtient 1,42 g sous forme d'une huile. Ensuite les énantiomères du couple de stéréoisomères (V+VIII) sont séparés sur une colonne CHIRALPAK^{®} AS (granulométrie 20 µm; diamètre 80 mm ; masse de la phase stationnaire 1,2 kg) sous une pression de 290 kPa, la phase mobile est composée d'un mélange de isopropanol-heptane-triéthylamine (10/90/0,1 en volumes) ayant un débit de 110 cm³ par minute et la longueur d'onde du détecteur UV est fixée à 265 nm. Les fractions contenant chaque énantiomère sont isolées puis concentrées sous une pression réduite (2 kPa) à une température voisine de 40°C ; c'est ainsi que l'on obtient 0,5 g du stéréoisomère V, les énantiomères du couple de stéréoisomères VI + VII sont séparés sur une colonne CHIRALCEL^{®} type OC (granulométrie 10 µm ; diamètre 60 mm ; masse de la phase stationnaire 600 g) sous une pression de 230 kPa, la phase mobile est composée d'un mélange éthanol-heptane-triéthylamine (10/90/0,1 en volumes) ayant un débit de 90 cm³ par minute et la longueur d'onde du détecteur UV est fixée à 265 nm. Les fractions contenant chaque énantiomère sont isolées puis concentrées sous une pression réduite (2 kPa) à une température voisine de 40°C ; c'est ainsi que l'on obtient 0,36 g du stéréoisomère VI, 0,68 g du stéréoisomère VII.

Spectre de R.M.N ¹H stéréoisomère V : (300 MHz, (CD₃)₂SO d6, δ en ppm) : de 0,80 à 2,00 (mt : 8H) ; 2,08 (dd large, J = 16,5 et 8 Hz : 1H); 2,35 (dd, J = 16,5 et 4 Hz : 1H) ; de 2,20 à 3,30 (mt : 8H) ; 3,55 (s : 3H) ; 3,92 (s : 3H) ; 5,33 (mt : 1H) ; 5,88 (d, J = 4 Hz : 1H) ; 7,08 (dd, J = 5,5 et 3,5 Hz : 1H) ; 7,25 (d large, J = 3,5 Hz : 1H) ; 7,42 (dd, J = 9 et 3 Hz : 1H); 7,68 (dd, J = 5,5 et 1 Hz : 1H); 7,95 (mt : 1H) ; 7,97 (d, J = 9 Hz : 1H) ; 8,69 (d large, J = 1,5 Hz : 1H) ; de 8,90 à 10,00 (mfs : 1H).
Condition HPLC : séparation sur phase Chiralpak AS 20µm.
Condition d'élution : heptane, éthanol, tréthylamine(85/15/0.1% volume) ; 1ml/min UV254nm
Temps de rétention : 9,37 min

Spectre de R.M.N ¹H stéréoisomère VI : (300 MHz, (CD₃)₂SO d6, δ en ppm) : de 1,00 à 2,65 (mt : 12H) ; 2,06 (dd, J = 15,5 et 7,5 Hz : 1H) ; 2,40 (dd, J = 15,5 et 4 Hz : 1H) ; de 2,65 à 2,90 (mt : 2H) ; 2,91 (t large, J = 7 Hz : 2H) ; 3,54 (s : 3H) ; 3,90 (s : 3H) ; 5,32 (mt : 1H) ; 5,84 (d, J = 4 Hz : 1H) ; 7,05 (dd, J = 5,5 et 3,5 Hz : 1H) ; 7,16 (dd, J = 3,5 et 1,5 Hz : 1H) ; 7,39 (dd, J = 9 et 3 Hz : 1H) ; 7,61 (dd, J = 5,5 et 1,5 Hz : 1H) ; 7,96 (d, J = 9 Hz : 1H) ; 7,99 (mt : 1H) ; 8,68 (d large, J = 1,5 Hz : 1H).

### Condition HPLC : séparation sur phase type Chiracel OC 10 µm

Condition d'élution : heptane, éthanol, triéthylamine (90/10/ 0.1 % en volume) ; 1 ml/min; UV 254 nm
Temps de rétention: 17,5 min

Spectre de R.M.N ¹H stéréoisomère VII : (300 MHz, (CD₃)₂SO d6, δ en ppm) : de 1,00 à 2,65 (mt : 12H) ; 2,05 (dd, J = 15 et 7,5 Hz : 1H) ; 2,40 (dd, J = 15 et 4 Hz : 1H) ; 2,79 (mt : 2H) ; 2,93 (t large, J = 7 Hz : 2H) ; 3,56 (s : 3H) ; 3,90 (s : 3H) ; 5,31 (mt : 1H); 5,84 (d, J = 4 Hz : 1H) ; 7,05 (dd, J = 5,5 et 3,5 Hz : 1H) ; 7,19 (d large, J = 3,5 Hz : 1H) ; 7,40 (dd, J = 9 et 3 Hz : 1H) ; 7,62 (dd, J = 5,5 et 1 Hz : 1H) ; 7,96 (d, J = 9 Hz : 1H) ; 7,99 (mt : 1H) ; 8,68 (d large, J = 1,5 Hz : 1H).

### Condition HPLC : séparation sur phase type Chiracel OC 10 µm

Condition d'élution : heptane, éthanol, triéthylamine (90/10/ 0.1 % en volume) ; 1 ml/min;
UV 254 nm.
Temps de rétention : 23,76 min

### Exemple 9

### Dichlorhydrate de l'acide (3RS,4RS)-4-[3-(R,S)-hydroxy-3-(3-fluoro-6-méthoxyquinolin-4-yl)propyl]-1-[2-(2-thiénylthio)éthyl]-pipéridine-3-carboxylique

Une solution de 630 mg de (3RS,4RS)-4-[3-(R,S)-hydroxy-3-(3-fluoro-6-méthoxyquinolin-4-yl)propyl]-1-[2-(2-thiénylthio)éthyl]-pipéridine-3-carboxylate de méthyle, 6 cm³ de dioxane et 3 cm³ d'une solution aqueuse d'hydroxyde de sodium 1N, est chauffée sous agitation à une température voisine de 60°C pendant 2 heures. 3cm³ d'une solution aqueuse d'hydroxyde de sodium 1N sont ajouté et la solution est chauffée 1 heure à 60°C. Après concentration du mélange réactionnel sous pression réduite (5 kPa) à une température voisine de 40°C, le résidu obtenu est repris par 20 cm³ d'eau et 20 cm³ d'éther diéthylique. La phase aqueuse est décantée puis acidifiée par une coulée de 6 cm³ d'acide chlorhydrique 1N. Le précipité blanc formé est extrait par 150cm³ d'acétate d'éthyle. La phase organique est lavée par 2 fois 10 cm³ d'une solution aqueuse saturée de chlorure de sodium et séchée sur sulfate de magnésium, filtrée, puis concentrée sous pression réduite (5 kPa) à une température voisine de 40°C. On obtient 499 mg d'acide (3RS,4RS)-4-[3-(R,S)-hydroxy-3-(3-fluoro-6-méthoxyquinolin-4-yl)propyl]-1-[2-(2-thiénylthio)éthyl]-pipéridine-3-carboxylique, sous forme d'un solide de verseur blanche.

Spectre de R.M.N. ¹H (300 MHz, (CD₃)₂SO d6 avec, δ en ppm). Nous observons un mélange de stéréoisomères dans les proportions 50/50.
* de 0,90 à 2,25 (mt : 10H) ; de 2,45 à 2,65 (mt : 2H) ; de 2,70 à 3,05 (mt : 4H) ; 3,92 (s : 3H) ; 5,31 (mt : 1H) ; de 5,50 à 6,20 (mf étalé : 1H) ; 7,04 (mt : 1H) ; 7,18 (mt : 1H) ; 7,38 (mt : 1H) ; 7,59 (mt : 1H) ; de 7,90 à 8,05 (mt : 2H) ; 8,67 (mt : 1H) ; de 11,50 à 13,50 (mf très étalé).

### (3RS,4RS)-4-[3-(R,S)-hydroxy-3-(3-fluoro-6-méthoxyquinolin-4-yl)propyl]-1-[2-(2-thiénylthio)éthyl]-pipéridine-3-carboxylate de méthyle

A une solution composée de 1,17 g de dichlorhydrate de (3RS, 4RS) -4-[3-(R,S)-hydroxy-3-(3-fluoro-6-méthoxyquinolin-4-yl)propyl]-pipéridine-3-carboxylate de méthyle dans 20 cm³ d'acétonitrile et 10 cm³ de DMF, on ajoute 0,74 cm³ de triéthylamine puis 638 mg de 2-(2-bromoéthylthio)thiophène dans 10cm³ d'acétonitrile, 1 g de carbonate de potassium, 431 mg d'iodure de potassium. Le mélange est agitée pendant 15 heures à une température voisine de 65°C sous atmosphère inerte. Après refroidissement à environ 20°C, le mélange réactionnel est filtré sur verre fritté. Le filtrat est repris par 100cm³ d'acétonitrile et concentré sous presion réduite (5 kPa). Le résidu est repris par 200 cm³ d'acétate d'éthyle et 100 cm³ d'eau. La phase organique est décantée, lavée par 2 fois 75 cm³ d'une solution aqueuse saturée de chlorure de sodium, séchée sur sulfate de magnésium, filtrée, concentrée sous pression réduite (5 kPa) à une température voisine de 40°C. Le résidu obtenu est purifié par chromatographie, sous une pression d'azote de 50 kPa, sur une colonne de gel de silice (granulométrie 20-45 µ; diamètre 2 cm; hauteur 40 cm), en éluant par un mélange de cyclohexane-acétate d'éthyle (25/75 en volumes) et en recueillant des fractions de 50 cm³. On concentre les fractions 6 à 8. On obtient 630 mg de (3RS,4RS)-4-[3-(R,S)-hydroxy-3-(3-fluoro-6-méthoxyquinolin-4-yl)propyl]-1-[2-(2-thiénylthio)éthyl]-pipéridine-3-carboxylate de méthyle, sous forme d'une huile incolore.

Spectre infra-rouge (CC14) : 3616 ; 2950 ; 2811 ; 2770 ; 1739 ; 1623 ; 1508 ; 1497; 1354 ; 1232 ; 1158; 1133 ; 1034 ; 907 ; 834 et 700 cm-1

### Dichlorhydrate de (3RS,4RS)-4-[3-(R,S)-hydroxy-3-(3-fluoro-6-méthoxyquinolin-4-yl)propyl]-pipéridine-3-carboxylate de méthyle

Une solution de 1,26 g d'acide (3RS,4RS)-4-[3-(R,S)-hydroxy-3-(3-fluoro-6-méthoxyquinolin-4-yl)propyl]-1-(tert-butyloxycarbonyl)-pipéridine-3-carboxylique dans 50 cm³ de méthanol est refroidie à une température voisine de -25°C, sous agitation et sous atmosphère inerte. A cette solution est ajoutée en 25 minutes 1,6 cm³ de chlorure de thionyle. Le mélange est ramené à une température voisine de 20°C, tandis que l'agitation est poursuivie pendant encore 48 heures. On concentre le mélange réactionnel sous pression réduite (5 kPa) à une température voisine de 40°C, reprend par 100 cm³ de toluène et concentre à nouveau le mélange sous pression réduite (5 kPa). Le résidu est repris par 50 cm³ d'oxyde de diisopropyle, trituré et concentré à sec sous presion réduite (5 kPa). On obtient 1,17 g de dichlorhydrate de (3RS,4RS)-4-[3-(R,S)-hydroxy-3-(3-fluoro-6-méthoxyquinolin-4-yl)propyl]-pipéridine-3-carboxylate de méthyle, sous forme d'une poudre jaune.

Spectre infra-rouge (comprimé KBr):3415; 3129; 2949; 2772; 2473; 2022; 1733; 1622; 1603; 1555; 1496; 1420; 1307; 1242; 1172; 1019; 871 et 795 cm-1

### Acide (3RS,4RS)-4-[3-(R,S)-hydroxy-3-(3-fluoro-6-méthoxyquinolin-4-yl)propyl]-1-(tert-butyloxycarbonyl)-pipéridine-3-carboxylique

Une solution de 1,77g de (3RS,4RS)-4-[3-(3-fluoro-6-méthoxyquinolin-4-yl)-3-propyl]-1-(tert-butyloxycarbonyl)-pipéridine-3-carboxylate de méthyle, 140 cm³ de diméthylsulfoxyde anhydre et 30 cm³ de tert-butanol anhydre est agitée sous atmosphère inerte exempte d'eau à 20°C. On purge cette solution avec de l'oxygène pur jusqu'à saturation du mélange réactionnel. On ajoute alors une solution contenant 1,75 g de tert-butoxyde de potassium dans 10 cm³ de tert-butanol anhydre. L'oxygène est de nouveau introduit par barbotage pendant encore 1 heure et 10 minutes sous forte agitation. La solution obtenue est purgée à l'azote puis refroidie à 0°C. On ajoute ensuite 1 cm³ d'acide acétique pur dans 100 cm³ d'eau puis 100 cm³ d'eau et 500 cm³ d'éther. La phase organique est décantée, lavée par 7 fois 100 cm³ d'eau et par 3 fois 100 cm³ d'une solution aqueuse saturée de chlorure de sodium. La phase aqueuse est extraite par 400 cm³ d'acétate d'éthyle, lavée par 7 fois 75 cm³ d'eau et 3 fois 75 cm³ d'une solution aqueuse saturée de chlorure de sodium. Les 2 phases organiques sont réunies, séchées sur du sulfate de magnésium, filtrées et concentrées à sec sous pression réduite (5kPa) à une température voisine de 40°C. On obtient 1,26 g d'acide (3RS,4RS)-4-[3-(R,S)-hydroxy-3-(3-fluoro-6-méthoxyquinolin-4-yl)propyl]-1-(tert-butyloxycarbonyl)-pipéridine-3-carboxylique sous forme d'une meringue blanche.

Spectre de R.M.N. ¹H (400 MHz, (CD₃)₂SO d6 avec, à une température de 383 K, δ en ppm). Nous observons un mélange de stéréoisomères.
* de 0,85 à 2,30 (mt : 16H) ; de 2,60 à 3,25 (mt : 5H); 3,88 (mt : 1H); 3,94 (s : 3H); 4,00 (dd large, J = 14 et 3 Hz : 1H); 5,35 (mt : 1H); 7,38 (mt : 1H); de 7,90 à 8,00 (mt : 2H) ; 8,63 (s large : 1H).

### (3RS,4RS)-4-[3-(3-fluoro-6-méthoxyquinolin-4-yl)-3-propyl]-1-(tert-butyloxycarbonyl)-pipéridine-3-carboxylate de méthyle

Une solution de 1,45 g de (3RS,4RS)-1-(tert-butyloxycarbonyl)-4-allyl-pipéridine-3-carboxylate de méthyle dans 20 cm³ de tétrahydrofurane est ajoutée lentement, à une température voisine de 0°C, sous agitation et sous atmosphère inerte, à 16 cm³ d'une solution 0,5 M de 9-borabicyclo[3,3,1]nonane dans le tétrahydrofurane. Le mélange est ensuite ramené à une température voisine de 20°C, tandis que l'agitation est poursuivie pendant encore 4 heures. 1,52 g de 4-iodo-3-fluoro-6-méthoxy quinoléine en solution dans 40 cm³ de tétrahydrofurane sont ajoutés, puis 100 mg de chlorure de palladium diphénylphosphinoferrocène et enfin 3,18 g de phosphate de potassium tribasique. Le mélange réactionnel est chauffé pendant 15 heures au reflux puis filtré à chaud sur verre fritté. Le filtrat est repris dans 50 cm³ d'acétate d'éthyle et concentré à sec sous pression réduite (5 kPa). Le résidu est repris par 75 cm³ d'acétate d'éthyle et 40 cm³ d'eau. La phase organique est décantée, lavée par 2 fois 40 cm³ d'une solution aqueuse saturée de chlorure de sodium, séchée sur sulfate de magnésium, filtrée, puis concentrée sous pression réduite (5 kPa) à une température voisine de 40°C. Le résidu est purifié par chromatographie, sous une pression d'azote de 50 kPa, sur une colonne de gel de silice (granulométrie 20-45 µ; diamètre 2,5 cm ; hauteur 40 cm), en éluant par un mélange de cyclohexane-acétate d'éthyle (75/25 en volumes) et en recueillant des fractions de 50 cm³. Les fractions 16 à 22 sont réunies, puis concentrées. On obtient 1,77 g de (3RS,4RS)-4-[3-(3-fluoro-6-méthoxyquinolin-4-yl)-3-propyl]-1-(tert-butyloxycarbonyl)-pipéridine-3-carboxylate de méthyle sous forme d'une huile incolore.

Spectre de R.M.N. ¹H (300 MHz, (CD₃)₂SO d6 avec, δ en ppm) : de 1,25 à 1,90 (mt : 16H) ; 2,61 (mt : 1H) ; de 2,65 à 3,25 (mt : 4H) ; 3,47 (s large : 3H) ; de 3,60 à 4,05 (mt : 2H) ; 3,96 (s : 3H) ; 7,36 (d, J = 3 Hz : 1H); 7,40 (dd, J = 9 et 3 Hz : 1H) ; 7,97 (d, J = 9 Hz : 1H) ; 8,70 (s large : 1H).

### (3RS,4RS)-1-(tert-butyloxycarbonyl)-4-allyl-pipéridine-3-carboxylate de méthyle

On ajoute 11,34 g d'hydrure de tributylétain à une solution de 10,1 g de 1-(tert-butyloxycarbonyl)-4-allyl-4-(méthoxallyl)-hydroxy-pipéridine-3-carboxylate de méthyle dans 250 cm³ de toluène sous atmosphère inerte et à une température proche de 20°C. On ajoute ensuite 25 mg d'AIBN et porte le mélange réactionnel au reflux du solvant pendant 1 heure. Le mélange est refroidi à une température proche de 20°C, concentré à sec sous pression réduite (5 kPa). Le résidu est repris par 50 cm³ de dichlorométhane puis purifié par chromatographie, sous une pression d'azote de 50 kPa, sur une colonne de gel de silice (granulométrie 20-45 µ ; diamètre 7 cm ; hauteur 40 cm), en éluant par un mélange de cyclohexane-acétate d'éthyle (75/25 en volumes) et en recueillant des fractions de 200 cm³ Les fractions 6 à 8 sont réunies, puis concentrées. On obtient 1,4 g de (3RS,4RS)-1-(tert-butyloxycarbonyl)-4-allyl-pipéridine-3-carboxylate de méthyle sous forme d'une huile incolore.

Spectre de R.M.N. ¹H (400 MHz, (CD₃)₂SO d6 avec, à une température de 353K, δ en ppm) : de 1,35 à 1,50 (mt : 1H); 1,40 (s : 9H); de 1,65 à 1,80 (mt : 1H) ; 1,89 (mt : 1H) ; de 1,95 à 2,20 (mt : 2H) ; 2,62 (mt : 1H) ; 2,97 (mt : 1H) ; 3,22 (dd, J = 14 et 4 Hz : 1H) ; 3,60 (s : 3H) ; de 3,65 à 3,80 (mt : 1H) ; 3,91 (dd large, J = 14 et 5 Hz : 1H) ; 5,02 (mt : 2H) ; de 5,65 à 5,85 (mt : 1H).

### 1-(tert-butyloxycarbonyl)-4-allyl-4-(méthoxallyl)-hydroxy-pipéridine-3-carboxylate de méthyle

On ajoute, sous atmosphère inerte, 7,57 g de diméthylaminopyridine à une solution de 9,4 g de 1-(tert-butyloxycarbonyl)-4-allyl-4-hydroxy-pipéridine-3-carboxylate de méthyle dans 120 cm³ d'acétonitrile puis on verse en 20 minutes 5,7 cm³ de chlorure d'oxalyle. Après 15 heures d'agitation à une température proche de 20°C, on ajoute 1,22 g de diméthylaminopyridine puis 0,92 cm³ de chlorure d'oxalyle. L'agitation est maintenue pendant 15 heures à la même température. On répète encore une fois ce processus d'addition et continue l'agitation 6 heures. Le mélange réactionnel est repris par 200 cm³ d'acétate d'éthyle et 200 cm³ d'une solution aqueuse saturée de bicarbonate de sodium. La phase organique est décantée, lavée par 2 fois 100 cm³ d'une solution aqueuse saturée de chlorure de sodium, séchée sur sulfate de magnésium, filtrée sur verre fritté. Le filtrat est agité pendant une heure avec 11 g de silice, filtré sur verre fritté, repris ave 20 g de silice et 300 cm³ d'acétate d'éthyle, puis concentrée sous pression réduite (5 kPa) à une température voisine de 40°C. On obtient 12,15 g de 1-(tert-butyloxycarbonyl)-4-allyl-4-(méthoxallyl)-hydroxypipéridine-3-carboxylate de méthyle sous forme d'une huile beige.

Spectre infra-rouge (CC14):3082; 2980; 2954; 1775; 1748; 1699; 1641; 1424; 1392; 1367; 1245; 1202; 1162; 1142; 993 et 925 cm-1

### 1-(tert-butyloxycarbonyl)-4-allyl-4-hydroxy-pipéridine-3-carboxylate de méthyle

On ajoute, sous atmosphère inerte et à une température proche de 20°C, 101,66 g de 1-(*tert*-butyloxycarbonyl)-4-oxo-pipéridine-3-carboxylate de méthyle à 2,156 litres d'une solution aqueuse saturée de chlorure d'ammonium et de THF (10/1 en volumes) de THF. On ajoute ensuite 34,73 cm³ de bromure d'allyle puis 77,51 g de zinc en maintenant la température inférieure à 30°C. On verse ensuite goutte à goutte une solution de 69,47 cm³ de bromure d'allyle dans 50 cm³ de THF. Après 3 heures d'agitation à une température proche de 20°C, la réaction est incomplète. On ajoute à nouveau 77,51 g de zinc puis on verse ensuite goutte à goutte 104 cm³ de bromure d'allyle en maintenant la température inférieure à 30°C. L'agitation est maintenue à une température proche de 20°C pendant 15 heures. La réaction est incomplète. On ajoute à nouveau 35 g de zinc puis on verse ensuite goutte à goutte 55 cm³ de bromure d'allyle en maintenant la température inférieure à 30°C. L'agitation est maintenue à une température proche de 20°C pendant 4 heures. La réaction est incomplète. On ajoute à nouveau 10 g de zinc puis on verse ensuite goutte à goutte 25 cm³ de bromure d'allyle en maintenant la température inférieure à 30°C. L'agitation est maintenue à une température proche de 20°C pendant 2 heures. Le mélange réactionnel est repris par 900 cm³ d'une solution HCl 1N et 2 litres d'éther éthylique et filtré sur verre fritté. Le filtrat est lavé par 3 fois 500 cm³ d'éther étylique. La phase organique est lavé par une solution aqueuse saturée en chlorure de sodium, séchée sur du sulfate de magnésium et concentrée à sec sous pression réduite (5 kPa). On obtient un huile jaune qui est purifiée par chromatographie, sous une pression d'azote de 50 kPa, sur une colonne de gel de silice (granulométrie 20-45 µ ; diamètre 12 cm ; hauteur 60 cm), en éluant par un mélange de cyclohexane-acétate d'éthyle (80/20 en volumes) et en recueillant des fractions de 200 cm³. Les fractions 51 à 75 sont réunies, puis concentrées. On obtient 63,5 g de 1-(tert-butyloxycarbonyl)-4-allyl-4-hydroxypipéridine-3-carboxylate de méthyle sous forme d'une huile jaune claire.

Spectre infra-rouge (CC14) :3513; 3078; 2980; 2954; 1697; 1641; 1423; 1392; 1366; 1200; 1163; 962 et 919 cm-1

### 1-(tert-Butyloxycarbonyl)-4-oxo-pipéridine-3-carboxylate de méthyle

A une suspension de 89,93 g de chlorhydrate de 3-méthoxycarbonyl-4-pipéridone (à 98 % de pureté) dans 900 cm³ de dichlorométhane, refroidie à une température voisine de 0°C, on ajoute sous agitation 65,27 cm³ de triétylamine, puis 91,12 g de di-tert-*butyl* dicarbonate préalablement solubilisés dans 400 cm³de dichlorométhane. Le mélange est agité pendant 12 heures à une température proche de 20°C. Le chlorhydrate de triétylamine est filtré, puis le filtrat est lavé par 3 fois 500 cm³ d'eau puis par 2 fois 500 cm³ d'une solution aqueuse saturée de chlorure de sodium. La phase organique est filtré sur fritté contenant de la silice fine, séchée sur sulfate de sodium, filtrée puis concentrée sous pression réduite (5 kPa) à une température voisine de 40°C. On obtient 101,66 g de 1-(*tert*-butyloxycarbonyl)-4-oxo-pipéridine-3-carboxylate de méthyle, sous forme d'une huile jaune claire.

Spectre infra rouge (CH₂Cl₂) : 2982 ; 2932 ; 2872 ; 1740 ; 1691 ; 1664 ; 1622 ; 1477 ; 1468 ; 1423 ; 1367 ; 1338 ; 1309 ; 1235 ; 1200 ; 1167 ; 1123 ; 1064. cm⁻¹

La présente invention concerne également les compositions pharmaceutiques contenant au moins un dérivé de quinolyl propyl pipéridine selon l'invention, le cas échéant sous forme de sel, à l'état pur ou sous forme d'une association avec un ou plusieurs diluants ou adjuvants compatibles et pharmaceutiquement acceptables.

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, les nouveaux dérivés de quinolyl propyl pipéridine selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 750 mg et 3 g de produit actif en 2 ou 3 prises par jour par voie orale ou entre 400 mg et 1,2 g par voie intraveineuse pour un adulte.

L'exemple suivant illustre une composition selon l'invention.

### EXEMPLE 1

On prépare selon la technique habituelle une composition liquide destinée à l'usage parentéral comprenant :
- dichlorhydrate de l'acide (3RS,4RS)-4-[3-(3-fluoro-6-méthoxyquinolin-4-yl)propyl]-1-[2-(thién-2-yl)thioéthyl]-pipéridine-3-acétique 125 mg
- glucose qsp 5%
- hydroxyde de sodium qsp pH = 4-4,5
- eau ppi qsp 50 ml

### EXEMPLE 2

On prépare selon la technique habituelle une composition liquide destinée à l'usage parentéral comprenant :
- dichlorhydrate de l'acide (3RS,4RS)-4-[3-(R,S)-hydroxy-3-(3-fluoro-6-méthoxyquinolin-4-yl)propyl]-1-[2-(2-thiénylthio)éthyl]-pipéridine-3-acétique 125 mg
- glucose qsp 5 %
- hydroxyde de sodium qsp pH = 4-4,5
- eau ppi qsp 50 ml

## Revendications

1. Un dérivé de quinolyl propyl pipéridine, **caractérisé en ce qu'**il répond à la formule générale 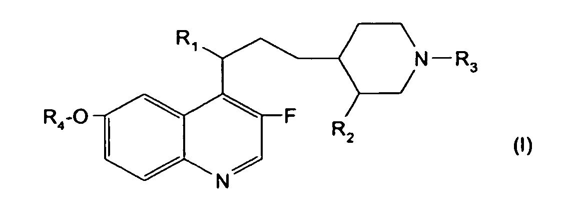 pour lesquels :
R₁ est un atome d'hydrogène ou d'halogène ou un radical hydroxy, amino, alcoylamino, dialcoylamino, hydroxyamino, alcoyloxyamino ou alcoyl alcoyloxy amino,
R₂ représente un radical carboxy, carboxyméthyle ou hydroxyméthyle,
R₃ représente un radical alcoyle (1 à 6 atomes de carbone) substitué par un radical phénylthio pouvant lui même porter 1 à 4 substituants [choisis parmi halogène, hydroxy, alcoyle, alcoyloxy, trifluorométhyle, trifluorométhoxy, carboxy, alcoyloxycarbonyle, cyano ou amino], par un radical cycloalcoylthio dont la partie cyclique contient 3 à 7 chaînons, ou par un radical hétérocyclylthio aromatique de 5 à 6 chaînons comprenant 1 à 4 hétéroatomes choisis parmi l'azote, l'oxygène ou le soufre et éventuellement lui même substitué [par halogène, hydroxy, alcoyle, alcoyloxy, trifluorométhyle, trifluorométhoxy, oxo, carboxy, alcoyloxycarbonyle, cyano ou amino] et
R₄ représente un radical alcoyle (contenant 1 à 6 atomes de carbone), alcényl-CH₂- ou alcynyl-CH₂- (dont les parties alcényle ou alcynyle contiennent 2 à 6 atomes de carbone), cycloalcoyle ou cycloalcoyl alcoyle (dont la partie cycloalcoyle contient 3 à 8 atomes de carbone)
étant entendu que les radicaux et portions alcoyle sont en chaîne droite ou ramifiée et contiennent (sauf mention spéciale) 1 à 4 atomes de carbone
sous ses formes diastéréoisomères ou leurs mélanges et/ou sous ses formes cis ou trans, ainsi que ses sels.

2. L'acide (3RS,4RS)-4-[3-(R,S)-hydroxy-3-(3-fluoro-6-méthoxyquinolin-4-yl)propyl]-1-[2-(2-thiénylthio)éthyl]-pipéridine-3-carboxylique, ainsi que ses sels.

3. Un procédé de préparation de dérivé de quinolyl propyl pipéridine selon la revendication 1, **caractérisé en ce que** l'on condense la chaîne R₃ définie dans la revendication 1, sur le dérivé de quinolyl propyl pipéridine de formule générale : 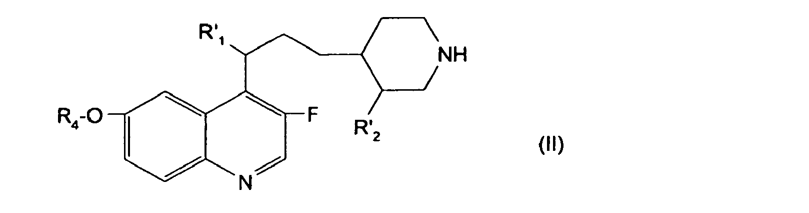 dans laquelle R₄ est défini comme dans la revendication 1, R'₁ représente un atome d'hydrogène ou un radical hydroxy et R'₂ représente un radical carboxy ou carboxyméthyle protégé, pour obtenir un dérivé de quinolyl propyl pipéridine de formule générale : 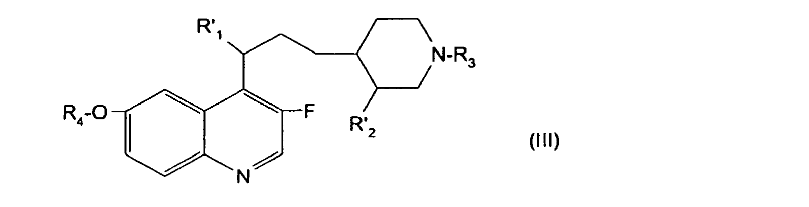 pour lequel R'₁, R'₂ et R₄ sont définis comme ci-dessus et R₃ est défini comme dans la revendication 1,
puis, le cas échéant, met en oeuvre l'halogénation du dérivé pour lequel R'₁ est un radical hydroxy, pour obtenir un dérivé de quinolyl propyl pipéridine pour lequel R₁ est un atome d'halogène,
ou bien, transforme le radical hydroxy en un radical oxo, puis en un radical hydroxyimino ou alcoyloxyimino, selon les métodes connues, pour obtenir un dérivé de quinolyl propyl pipéridine de formule générale : 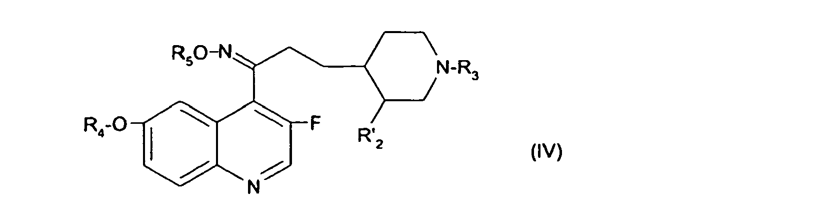 pour lequel R'₂ est défini comme ci-dessus, R₃ et R₄ sont définis comme dans la revendication 1, et R₅ est un atome d'hydrogène ou un radical alcoyle, et réduit le dérivé de formule générale (IV) pour lequel R₅ est un atome d'hydrogène en amine, et éventuellement transforme en une amine monoalcoylée ou dialcoylée, ou éventuellement de la réduit le dérivé de formule générale (IV) pour lequel R₅ est un atome d'hydrogène en hydroxylamine, ou le dérivé de formule générale (IV) pour lequel R₅ est un radical alcoyle en alcoyloxyamine, puis, le cas échéant, pour obtenir le dérivé pour lequel R₁ est alcoyl alcoyloxy amino, transforme le dérivé obtenu pour lequel R₁ est alcoyloxyamino, par alcoylation,
et/ou le cas échéant réduit le radical carboxy protégé R'₂ en un radical hydroxyméthyle selon les méthodes connues,
puis éventuellement sépare les diastéréoisomères, sépare les formes cis et trans, élimine le cas échéant le radical protecteur d'acide, et/ou transforme le produit obtenu en un sel.

4. Un procédé selon la revendication 3, **caractérisé en ce que** la condensation de la chaîne R₃ sur la pipéridine s'effectue par action d'un dérivé de formule générale:
R₃-X
dans laquelle R₃ est défini comme dans la revendication 1 et X représente un atome d'halogène, un radical méthylsulfonyloxy, un radical trifluorométhylsulfonyloxy ou p.toluènesulfonyloxy.

5. Un dérivé de quinolyl propyl pipéridine de formule générale : 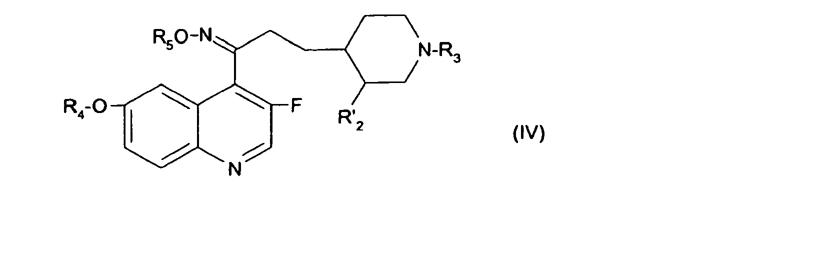 dans laquelle R'₂ est défini comme dans la revendication 3, R₃ et R₄ sont définis comme dans la revendication 1, et R₅ est un atome d'hydrogène ou un radical alcoyle.

6. Un dérivé de quinolyl propyl pipéridine de formule générale : 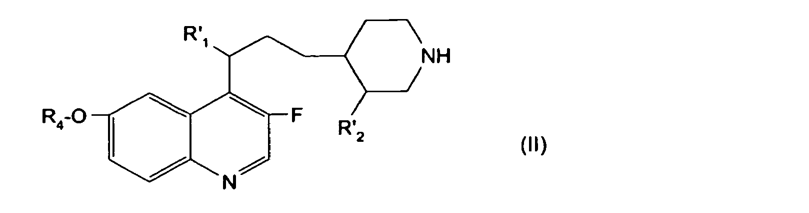 dans laquelle R'₁ et R'₂ sont définis comme dans la revendication 3 et R₄ est défini comme dans la revendication 1.

7. Un dérivé de quinolyl propyl pipéridine de formule générale : 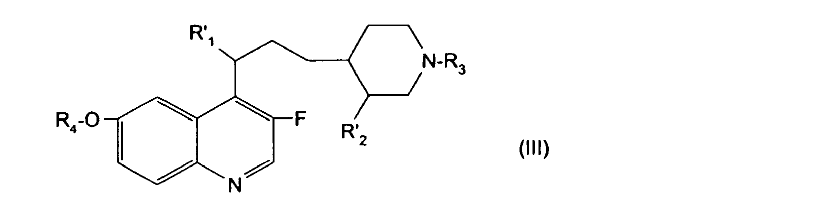 dans laquelle R'₁ et R'₂ sont définis comme dans la revendication 3 et R₃ et R₄ sont définis comme dans la revendication 1

8. Composition pharmaceutique **caractérisée en ce qu'**elle contient au moins un dérivé selon la revendication 1, à l'état pur ou en association avec un ou plusieurs diluants ou adjuvants compatibles et pharmaceutiquement acceptables.

## Claims

1. Quinolylpropylpiperidine derivative,
**characterized in that** it corresponds to the general formula 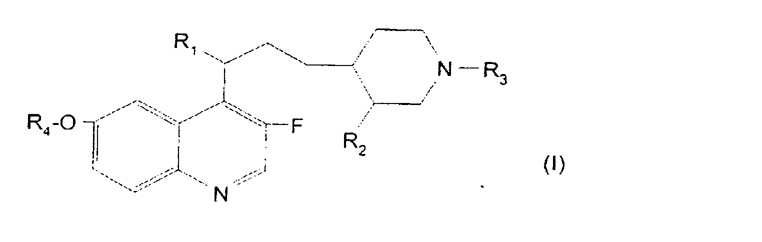 for which:
R₁ is a hydrogen or halogen atom or a hydroxyl, amino, alkylamino, dialkylamino, hydroxyamino, alkyloxyamino or alkylalkyloxyamino radical,
R₂ represents a carboxyl, carboxymethyl or hydroxymethyl radical,
R₃ represents an alkyl (1 to 6 carbon atoms) radical substituted with a phenylthio radical which may itself carry 1 to 4 substituents [chosen from halogen, hydroxyl, alkyl, alkyloxy, trifluoromethyl, trifluoro-methoxy, carboxyl, alkyloxycarbonyl, cyano or amino], with a cycloalkylthio radical in which the cyclic portion contains 3 to 7 members, or with a 5- to 6-membered aromatic heterocyclylthio radical comprising 1 to 4 heteroatoms chosen from nitrogen, oxygen or sulfur and itself optionally substituted [with halogen, hydroxyl, alkyl, alkyloxy, trifluoromethyl, trifluoro-methoxy, oxo, carboxyl, alkyloxycarbonyl, cyano or amino], and
R₄ represents an alkyl (containing 1 to 6 carbon atoms), alkenyl-CH₂- or alkynyl-CH₂- (in which the alkenyl or alkynyl portions contain 2 to 6 carbon atoms), cycloalkyl or cycloalkylalkyl (in which the cycloalkyl portion contains 3 to 8 carbon atoms) radical,
it being understood that the alkyl radicals and portions are in the form of a straight or branched chain and contain (unless otherwise stated) 1 to 4 carbon atoms
in its diastereoisomeric forms or mixtures thereof and/or in its cis or trans forms, as well as its salts.

2. (3RS, 4RS)-4-[3-(R,S)-Hydroxy-3-(3-fluoro-6-methoxyquinolin-4-yl)propyl]-1-[2-(2-thienylthio) ethyl] piperidine-3-carboxylic acid, as well as its salts.

3. Process for preparing the quinolylpropylpiperidine derivative according to Claim 1, **characterized in that** the chain R₃ defined in Claim 1 is condensed with the quinolylpropylpiperidine derivative of general formula: 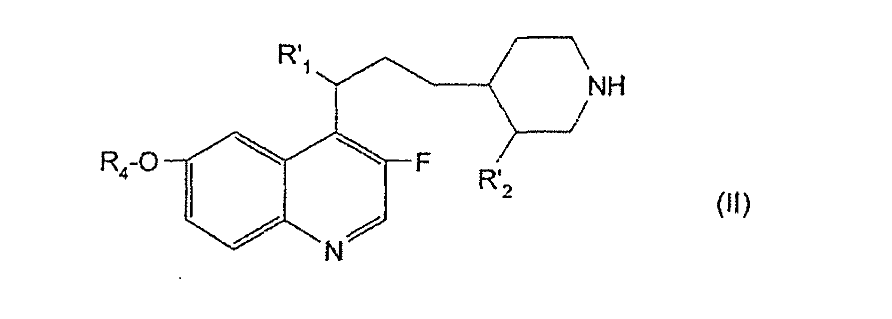 in which R₄ is as defined in Claim 1, R'₁ represents a hydrogen atom or a hydroxyl radical and R'₂ represents a protected carboxyl or carboxymethyl radical, to obtain a quinolylpropylpiperidine derivative of general formula: 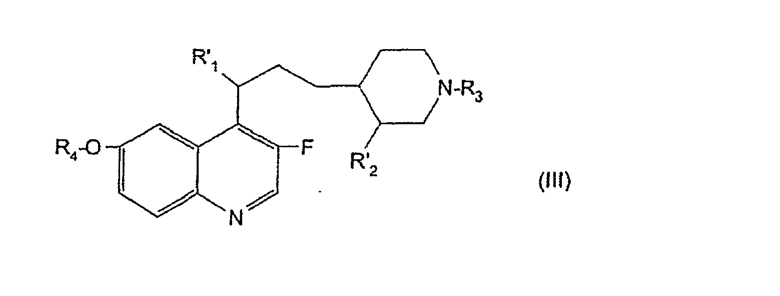 for which R'₁, R'₂ and R₄ are as defined above and R₃ is as defined in Claim 1,
and then, where appropriate, the derivative for which R'₁ is a hydroxyl radical is halogenated so as to obtain a quinolylpropylpiperidine derivative for which R₁ is a halogen atom,
or, the hydroxyl radical is converted to an oxo radical, and then to a hydroxyimino or alkyloxyimino radical, according to known methods, to obtain a quinolylpropylpiperidine derivative of general formula: 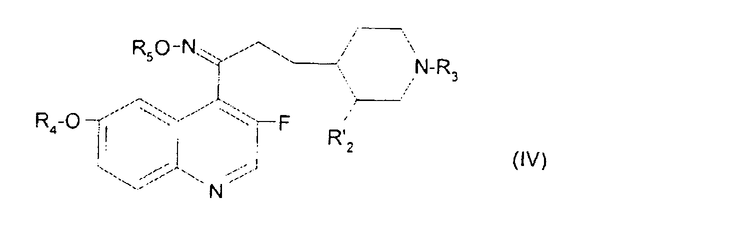 for which R'₂ is as defined above, R₃ and R₄ are as defined in Claim 1, and R₅ is a hydrogen atom or an alkyl radical, and the derivative of general formula (IV) for which R₅ is a hydrogen atom is reduced to an amine, and optionally converted to a monoalkylated or dialkylated amine, or optionally reduces the derivative of general formula (IV) for which R₅ is a hydrogen atom to a hydroxylamine, or the derivative of general formula (IV) for which R₅ is an alkyl radical to an alkyloxyamine, and then, where appropriate, to obtain the derivative for which R₁ is alkylalkyloxyamino, the derivative obtained for which R₁ is alkyloxyamino is converted by alkylation,
and/or, where appropriate, the protected carboxyl radical R'₂ is reduced to a hydroxymethyl radical according to known methods,
and then optionally the diastereoisomers are separated, the cis and trans forms are separated, the acid-protecting radical is eliminated, where appropriate, and/or the product obtained is converted to a salt.

4. Process according to Claim 3,
**characterized in that** the condensation of the chain R₃ with the piperidine is carried out by the action of a derivative of general formula:
R₃-X
in which R₃ is as defined in Claim 1 and X represents a halogen atom, a methylsulfonyloxy radical, a trifluoromethylsulfonyloxy or p-toluenesulfonyloxy radical.

5. Quinolylpropylpiperidine derivative of general formula: 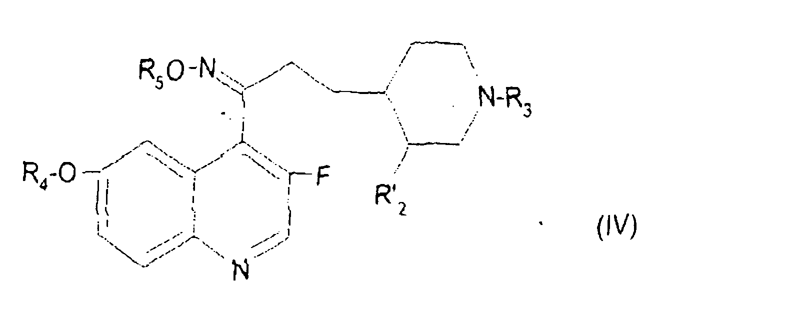 in which R'₂ is as defined in Claim 3, R₃ and R₄ are as defined in Claim 1, and R₅ is a hydrogen atom or an alkyl radical.

6. Quinolylpropylpiperidine derivative of general formula: 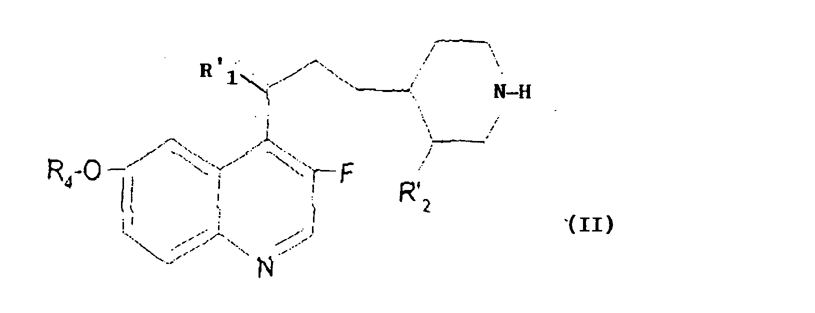 in which R'₁ and R'₂ are as defined in Claim 3, and R₄ is as defined in Claim 1.

7. Quinolylpropylpiperidine derivative of general formula: 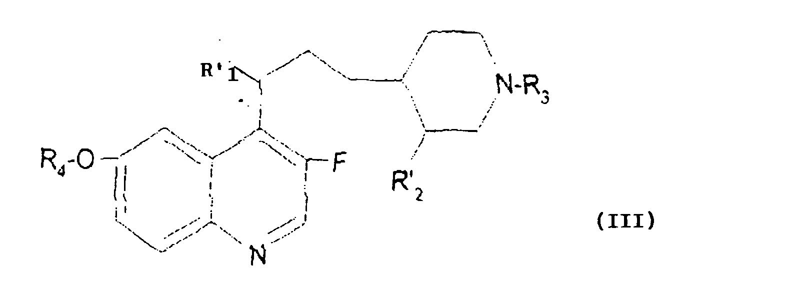 in which R'₁ and R'₂ are as defined in Claim 3 and R₃ and R₄ are as defined in Claim 1.

8. Pharmaceutical composition,
**characterized in that** it contains at least one derivative according to Claim 1, in the pure state or in combination with one or more compatible and pharmaceutically acceptable diluents or adjuvants.

## Patentansprüche

1. Derivat des Chinolylpropylpiperidins, **dadurch gekennzeichnet, dass** es der folgenden allgemeinen Formel entspricht 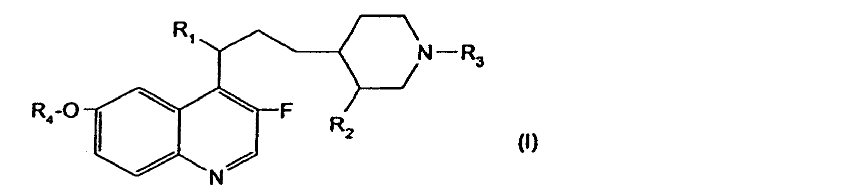 wobei:
R₁ für ein Wasserstoff- oder Halogenatom oder ein Hydroxy- Amino-, Alkylamino, Dialkylamino-, Hydroxyamino-, Alkyloxyamino- oder Alkylalkyloxyaminoradikal steht,
R₂ für ein Carboxy-, Carboxymethyl- oder Hydroxymethylradikal steht,
R₃ für ein Alkylradikal (1 bis 6 Kohlenstoffatome) steht, welches mit einem Phenylthioradikal, das seinerseits 1 bis 4 Substituenten [gewählt aus Halogen, Hydroxy, Alkyl, Alkyloxy, Trifluormethyl, Trifluormethoxy, Carboxy, Alkyloxycarbonyl, Cyano oder Amino] aufweisen kann, substituiert ist, oder welches mit einem Cycloalkylthioradikal dessen zyklischer Teil 3 bis 7 Glieder enthält, oder mit einem aromatischen heterozyklischen Thioradikal mit 5 bis 6 Gliedern, das 1 bis 4 Heteroatome, die aus Stickstoff, Sauerstoff oder Schwefel gewählt werden, umfasst und das möglicherweise seinerseits [mit Halogen, Hydroxy, Alkyl, Alkyloxy, Trifluormethyl, Trifluormethoxy, Oxo, Carboxy, Alkyloxycarbonyl, Cyano oder Amino] substituiert ist, und
R₄ für ein Alkyl- (1 bis 6 Kohlenstoffatome enthaltend), Alkenyl-CH₂- oder Alkinyl-CH₂- (deren Alkenyl- oder Alkinylgruppen 2 bis 6 Kohlenstoffatome enthalten) oder ein Cycloalkyl- oder Cycloalkylalkylradikal (dessen Cycloalkylgruppe 3 bis 8 Kohlenstoffatome enthält) steht
wobei die Ausführungen derart aufzufassen sind, dass die Alkylradikale und - gruppen als unverzweigte oder verzweigte Kette vorliegen und (wenn nichts anderes angegeben ist) 1 bis 4 Kohlenstoffatome enthalten
in Form seiner Stereoisomere oder deren Mischungen und/oder in seiner cis- oder trans-Form sowie in Form seiner Salze.

2. (3RS,4RS)-4-[3-(R,S)-Hydroxy-3-(3-fluor-6-methoxychinolin-4-yl)propyl]-1-[2-(2-thienylthio)ethyl]-piperidin-3-carbonsäure sowie deren Salze.

3. Verfahren zur Herstellung des Derivates des Chinolylpropylpiperidins gemäß dem Anspruch 1, **dadurch gekennzeichnet, dass** die Kette R₃, die in Anspruch 1 definiert ist, mittels einer Kondensationsreaktion in das Chinolylpropylpiperidinderivat, welches der folgenden allgemeinen Formel entspricht, eingeführt wird: 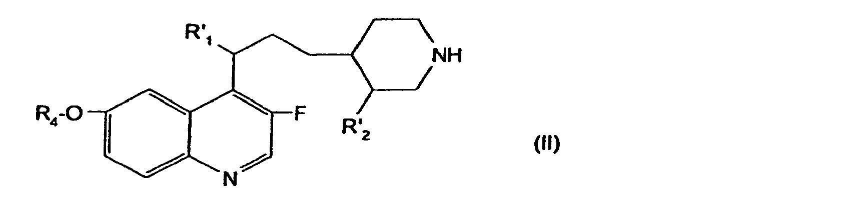 wobei R₄ der Definition in Anspruch 1 entspricht, während R'₁, für ein Wasserstoffatom oder ein Hydroxyradikal steht und R'₂ für ein geschütztes Carboxy- oder Carboxymethylradikal steht, woraufhin ein Chinolylpropylpiperidinderivat, welches der folgenden allgemeinen Formel entspricht, erhalten wird: 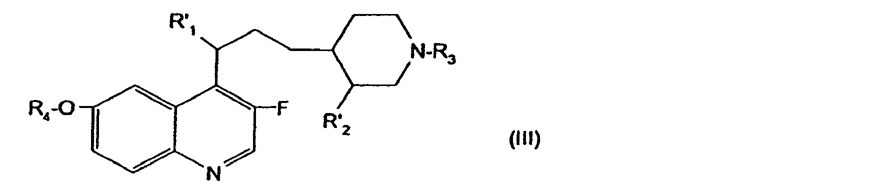 wobei R'₁, R'₂ und R₄ den obigen Definitionen entsprechen und R₃ der Definition in Anspruch 1 entspricht,
und dass gegebenenfalls das Derivat, bei welchem R'₁ für ein Hydroxyradikal steht, einer Halogenierung unterzogen wird, um ein Chinolylpropylpiperidinderivat zu erhalten, bei welchem R₁ für ein Halogenatom steht,
oder aber dass das Hydroxyradikal, gemäß bekannten Verfahren, zunächst in ein Oxoradikal und anschließend in ein Hydroxyimino- oder Alkyloxyiminoradikal umgewandelt wird, um ein Chinolylpropylpiperidinderivat, welches der folgenden allgemeinen Formel entspricht, zu erhalten: 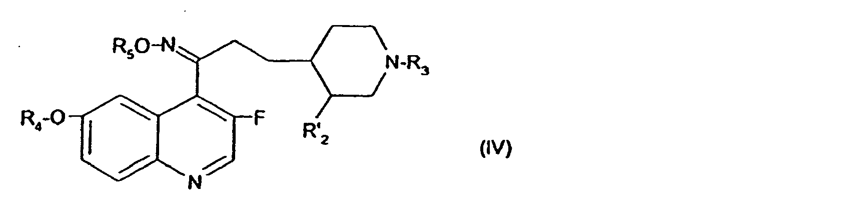 wobei R'₂ der obigen Definition entspricht, während R₃ und R₄ der Definition in Anspruch 1 entsprechen und R₅ für ein Wasserstoffatom oder ein Alkylradikal steht, woraufhin das Derivat nach der allgemeinen Formel (IV), bei welchem R₅ für ein Wasserstoffatom steht, zu einem Amin reduziert wird, woraufhin sich möglicherweise eine Umwandlung in ein monoalkyliertes oder dialkyliertes Amin anschließt, oder es erfolgt möglicherweise, ausgehend von dem Derivat nach der allgemeinen Formel (IV), bei welchem R₅ für ein Wasserstoffatom steht, eine Reduktion zu einem Hydroxylamin oder, ausgehend von dem Derivat nach der allgemeinen Formel (IV), bei welchem R₅ für ein Alkylradikal steht, eine Reduktion zu einem Alkyloxyamin, woraufhin gegebenenfalls das erhaltene Derivat, bei dem R₁ für Alkyloxyamino steht, durch Alkylierung umgewandelt wird, um das Derivat herzustellen, bei welchem R₁ für Alkylalkylamino steht,
und/oder das geschützte Carboxyradikal R'₂ wird gegebenenfalls gemäß bekannten Verfahren zu einem Hydroxymethylradikal reduziert,
woraufhin möglicherweise die Diastereoisomere voneinander getrennt werden, die cis- und trans-Formen voneinander getrennt werden und gegebenenfalls das Schutzradikal der Säure eliminiert wird und/oder das erhaltene Produkt in ein Salz überführt wird.

4. Verfahren gemäß dem Anspruch 3, **dadurch gekennzeichnet, dass** die Kondensation der Kette R₃ am Piperidin durch Einwirken eines Derivats erfolgt, welches der folgenden Formel entspricht:
R₃-X
wobei R₃ der Definition in Anspruch 1 entspricht und X für ein Halogenatom, ein Methylsulfonyloxyradikal, ein Trifluormethylsulfonyloxy- oder ein p.Toluolsulfonylradikal steht.

5. Derivat des Chinolylpropylpiperidins, welches der folgenden allgemeinen Formel entspricht: 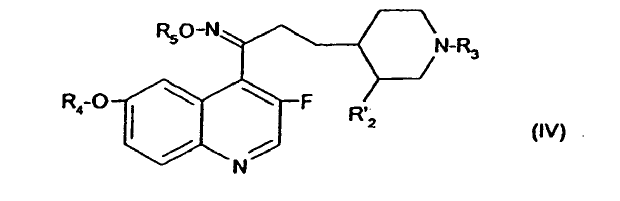 wobei R'₂ der Definition in Anspruch 3 entspricht, während R₃ und R₄ der Definition in Anspruch 1 entsprechen und es sich bei R₅ um ein Wasserstoffatom oder ein Alkylradikal handelt.

6. Derivat des Chinolylpropylpiperidins, welches der folgenden allgemeinen Formel entspricht: 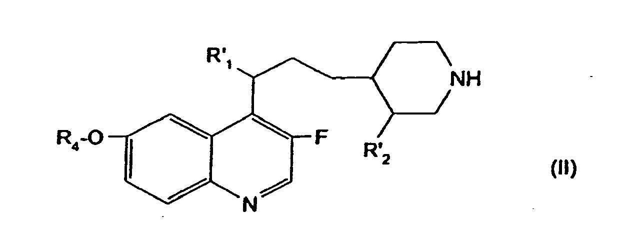 wobei R'₁ und R'₂ der Definition in Anspruch 3 entsprechen und R₄ der Definition in Anspruch 1 entspricht.

7. Derivat des Chinolylpropylpiperidins, welches der folgenden allgemeinen Formel entspricht: 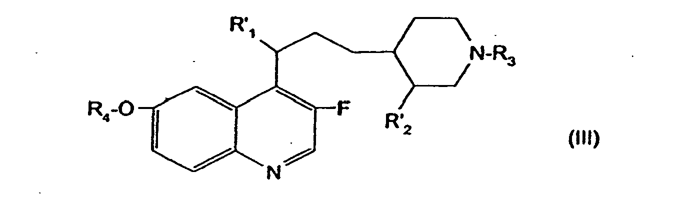 wobei R'₁ und R'₂ der Definition in Anspruch 3 entsprechen, während R₃ und R₄ der Definition in Anspruch 1 entsprechen.

8. Pharmazeutische Zusammensetzung, **dadurch gekennzeichnet, dass** sie mindestens ein Derivat gemäß dem Anspruch 1 enthält, und zwar in reinem Zustand oder in Verbindung mit einem oder mehreren Verdünnungsmitteln oder Hilfsstoffen, die verträglich und pharmazeutisch unbedenklich sind.
